# EUROPEAN PATENT APPLICATION

(11) **EP 1 792 627 A1**
(43) Date of publication of application: **06.06.2007**
(21) Application number: 05026476.1
(22) Date of filing: 05.12.2005
(51) Int. Cl.: A61K 39/35, A61P 37/08

(54) **Modulation of the immune response by administration of intralymphatic transduction allergen (ITAG-)-molecules**

(71) Applicant: ImVisioN AG, 30625 Hannover (DE)
(72) Inventor: Kündig, Thomas, Dr.med., 8303 Bassersdorf (CH); Steiner, Martin, Dr., 82061 Neuried (DE); Crameri, Reto, Prof.Dr., 7270 Davos-Platz (CH); Rose, Horst, Prof.Dr., 31303 Burgdorf (DE)
(74) Representative: Kröncke, Rolf

(57) **Abstract**

The present invention relates to the field of allergy vaccines and treatments. In particular, the invention provides new methods for desensitizing individuals to an allergen against which the individuals mount an allergic response. Furthermore, the invention relates to the use of specific molecules for the preparation of a medicament, in particular, for the preparation of a vaccine for intralymphatic injection for desensitisation of individuals and corresponding methods and kits comprising said compounds.

## Description

The present invention relates to the field of allergy vaccines and treatments. In particular, the invention provides new methods for desensitizing individuals to an allergen against which the individuals mount an allergic response. Furthermore, the invention relates to the use of specific molecules for the preparation of a medicament, in particular, for the preparation of a vaccine for intralymphatic injection for desensitisation of individuals and corresponding methods and kits comprising said compounds.

### Background

### Allergy

An allergy is the result of a powerful immune reaction against a substance, which is normally inoffensive to a host. That is, allergies are caused by an imbalance or hypersensitivity of the immune system, leading to a misdirected immune response. Typically, an allergic response occurs when the immune system reacts inappropriately to highly specific agents that in most people do not cause an immune response. Typical allergens known in the art are tree and grass pollen, cockroaches, dust mites, animal dander, latex, or insect venoms, like honeybee, wasp and fire ant venoms. Other common allergens include substances present in certain foods such as peanuts, tree nuts, milk, fish, shellfish, eggs, soy, wheat, honey, strawberries and tropical fruits, drugs such as penicillin and some anesthetics, serum, some viruses, bacteria and protozoa, and molds. Delayed type hypersensitivity reactions may occur in response to urushiol, an oil found in poison ivy, poison oak and sumac, resulting in a severe itchy rash and formation of oozing blisters.

### Allergens

An overview about allergens is given in the following documents, which are incorporated herein by reference (www.allergome.org; Brusil et al., Allergy, 2003, 58 :1093-1100). Usually, the first exposure to an allergen which is present in an allergen containing composition, like pollen, venom or food, causes only a mild immune response that sensitizes the immune system to the allergen. However, subsequent exposures to said allergen result in allergic symptoms, typically in a dose dependent manner and may cause an increasingly severe response with repeated exposures. The type of an allergic reaction and the symptoms depend on the specific allergen, the part of the body where exposure occurs, and the degree of sensitization of the individual.

Most people suffering from allergy are those who are allergic to pollen and are afflicted with airway diseases such as allergic rhinitis, hay fever and asthma. People with seasonal pollen allergies often develop cross-sensitivity to other allergens that are present all year, such as allergens derived from house dust (dust mite excrements). People with chronic respiratory allergies often develop asthma, which is the consequence of long-term activation of the allergic/inflammatory response in the respiratory system. The symptoms of asthma include coughing, wheezing, and shortness of breath due to a narrowing of the bronchial passages, excess mucus production and inflammation. Asthma can be disabling and sometimes fatal.

Cockroach allergy is an allergy to the excrement of cockroaches, and is a trigger of asthmatic attacks. Also the dust mite allergy is based on an allergy to the excrement of the dust mite which lives in dust found in all dwellings and workplaces, and in virtually all bedding. Dust mites are perhaps the most common cause of perennial allergic rhinitis, producing symptoms similar to pollen allergy and asthma. About half of all allergy sufferers are allergic to dust mites.

Over 10 million Americans are allergic to animals. Household pets are the most common source of such reactions. However, the major household pet allergens are not derived from the coat of these animals, but are proteins secreted by oil glands in the animal's skin and shed in dander and in the saliva, which sticks to the coat when the animal licks itself. When the saliva carrying the proteins dries, the proteins float into the air and are inhaled by people. Thus, exposure to airborne particles originating from household cats is also a common cause of allergic diseases. In particular, the Fel d 1 protein causes an allergic response in individuals having cat dander allergy. Some rodents like mice and rats as well as guinea pigs and gerbils, which have become increasingly popular as household pets can also cause allergic reactions in some people.

Another major cause of allergic reactions is food. Food allergies are different from food intolerance, since food intolerance does not involve an aberrant immune response. In particular, eight foods account for 90 % of food allergies in Americans effected by food allergies: milk, fish, peanuts, tree nuts, eggs, soy, wheat, and shellfish. Even in minute amounts the above food allergens may cause severe systemic reactions, such as onset of anaphylactic shock (allergic shock). Thus, individuals afflicted with allergy against certain food have to totally avoid such food. Conventional subcutaneous allergy shots are ineffective against food allergies.

Another major part of allergic people are individuals being allergic to bee or wasp stings. This type of allergy may result in serious consequences including death and the majority of individuals stung will experience a severe reaction if stung again.

It is important to distinguish between the term allergen as commonly used by the people in its broader sense and the allergen itself. That is, typically people denote e.g. whole birch pollen as the allergen. However, only few epitopes of a limited number of proteins represents the actual allergen, i.e. the allergy inducing element. With respect to birch pollen, which is an aggregation of various compounds, like proteins, sugars, lipids etc, only few proteins (e.g. the protein Bet v 1) contain an allergenic element, Bet v 1 and, thus, represent the allergen. The same is true for cat dander, which contain the Fel d 1 protein representing the protein containing the allergenic determinant, for bee venom wherein the molecule PLA 2 is one of the major proteins containing the allergenic element, for house dust, which is a material containing among many other substances the house dust mite excrements, containing i.a. the allergens Der p 1 and Der p 2.

Thus, it is necessary to differentiate between the term allergen as commonly used by the people denoting the materials in its totality, in the following also referred to as allergen containing composition, composition containing allerger(s) or allergen containing material, and the allergen in it's more narrow sense meaning the protein or lipid or other structure actually representing the allergenic determinant, i.e. the allergen.

### Allergic symptoms

Allergic symptoms normally start with itching and swelling of the affected tissues, rashes, muscle spasms and other more severe symptoms. Allergens that are inhaled often cause nasal congestion, itchy nose and throat, and mucus production. In highly allergic individuals or with higher doses of allergen, coughing, wheezing, or similar symptoms may occur. An ingested allergen may cause itching of the throat, vomiting, gastrospasm, diarrhea and skin rashes or shock, in cases of strong sensitivity. In fact, anaphylaxis, and in particular an anaphylactic shock represents the most dangerous symptom which may occur after an allergic reaction in an individual and may eventually lead to the death of the individual. Further, eczema is also associated with allergies; a decrease in allergies results in an improvement of eczema.

In table 1 below an overview of the classification of allergic reactions according to Müller is provided.

**Table 1 Müller Classification of Allergic Reactions**

| | |
|---|---|
| Large local reactions | Swelling at site of contact with allergen with diameter > 10 cm, lasting > 24h |

| Systemic reactions | |
|---|---|
| Grade I: | Generalized urticaria, itching, malaise, anxiety |
| Grade II: | Any of the above, plus 2 more of the following: angioedema (also grade II if occurs alone), constriction in chest, nausea, vomiting, diarrhea, abdominal pain, dizziness |
| Grade III: | Any of the above, plus 2 or more of the following: dyspnea, wheezing, stridor (any of these alone are grade III), dysphagia, dysarthria, hoarseness, weakness, confusion |
| Grade IV: | Any of the above, plus 2 or more of the following: fall in blood pressure, collapse, loss of consciousness, incontinence (urine, stool), cyanosis |

### Frequency of allergic diseases

In recent years, the frequency of allergic diseases has increased dramatically. For example, in the United States about 15 to 28 % of the population has allergies to some common substances (Ann Allergy Asthma Immunol, 1999, 82:233-248). During the 1970s the prevalence of allergic rhinitis and asthma among Swedish recruits roughly doubled from 1.9 to 2,8 % for asthma and from 4.4 to 8.4% for allergic rhinitis (Clin Exp Allergy, 1989, 19:59-63). Also in Swedish school children the prevalence of asthma, allergic rhinitis and eczema doubled between 1979 and 1991 with a prevalence of one or more symptoms of 23.8 to 44.8 % of all school children (Clin Exp Allergy, 1995, 25:815-19). In Denmark the prevalence of rhinitis in children of age 7 to 17 rose from 15 to 22% (Allergy, 2000, 55:1019-1024) and for adults of age 15 to 41 rose from 25 to 32% (J Allergy Clin Immunol 2000, 106:247-252). Heredity, environmental conditions, type and number of exposures, and various physiological factors such as stress, fatigue and emotional upset can increase the sensitivity of the immune system and predispose a person to allergies.

The reason for the increase in the number of allergy sufferers is not yet resolved and still under intense scientific debate. Various explanations have been discussed. For example, air pollution with nitric oxides may play a role in the increasing frequency of allergic airway disease, since they may directly damage sensitive cells lining the airway of the throat and lungs. This damage may allow more allergens to get into the body through damaged cells that normally protect the body from invasion by the allergen. In particular, it was shown that smog particles become adsorbed to pollen and act as an adjuvant, enhancing the effects of the allergens.

Another phenomenon described as cross-reactivity may also play a major role in the increasing number of individuals afflicted with allergy. Cross-reactivity occurs when an individual which reacts allergic to a particular allergen also has increased sensitization to another similar kind of allergen. Food allergies are commonly found to be associated with allergic airway diseases. For example, if the pollen of the hazelnut tree is inhaled, a person may develop an allergy to hazelnuts. In fact, cross-reactivity between allergens from pollen and food allergens may be one of the major causes of food allergies in adults. Another example of cross reactivity is known for the allergen Bet v1, the major birch pollen allergen and Api g1, a major apple allergen.

### Medical treatment of allergic diseases

In light of the increasing social and economic impact of allergies, treating and preventing allergic diseases has become a medical undertaking of growing importance. Typically, the following general approaches to help people with allergies are prosecuted:
a) avoidance of the allergen as much as possible;
b) medication to relieve allergic symptoms, and
c) desensitisation of individuals by administering a series of allergy shots.
Today several anti-allergy drugs exist. Unfortunately, these anti allergy drugs merely treat the symptoms of the allergies, have the risk of serious side effects and do not have any preventive or curative effect.

Another strategy is to develop ways of conditioning the immune system to respond "appropriately" to allergens. By way of prophylactic allergen immunotherapy of allergic people a causative treatment for allergies is possible.

Allergen immunotherapy or hyposensitization therapy (or "allergy shots") is a practice of administering gradually increasing quantities of an allergen to an allergic subject to ameliorate the symptoms (the allergic reaction) associated with subsequent exposure to the causative allergen. This type of allergen immunotherapy was,first conduced in 1911 and is currently established as the preferred treatment in the case of severe allergies.

Allergy shots sometimes allow significantly and permanently relieving the extent of suffering experienced by allergic individuals. Actually, the allergy shot approach is the only method that may be regarded as a curing means to prevent allergic reactions in individuals. Early desensitisation using the allergy shot approach to specific allergens has also shown some effect against the occurrence of cross-reactive allergies to other substances.

Although allergy shots are currently the only means for treating the disease rather than alleviating the symptoms, there are many disadvantages to this treatment as it is performed today. Usually, conventional immunotherapy is a long lasting procedure, requiring 30 to 100 allergen injections over a period of 2 to 5 years each requiring at least 1 hour under strict medical supervision after the shot is administered. Usually, the occurrence of the side effects is within 20 minutes, although some can develop up to 2 hours after the allergy shot is given. Consequently desensitisation therapy is very expensive.

In addition, allergy shot treatment is totally ineffective in one-third of allergic sufferers and only temporarily effective in one-third of allergic individuals. Immunotherapy has long term effectiveness in only the remaining third of the allergic population.

In some types of therapy (rush and ultra-rush) against insect venom allergy hospitalization of the allergic individual for several days is necessary, due to the increased risk of severe adverse events. Thus, medical and economic costs are even higher for this type of treatment.

Typically, allergy shot regimens involve two treatment phases. During the first phase the amount of allergen injected is increased with each dose, starting with very low amounts. The allergen containing composition or the pure allergen itself is normally diluted in organic solvents which may additionally contain stabilizers, e.g. phenol in an amount of 5 % w/v of the solution for injection. However, as it is commonly known, organic solvents and stabilizing agents, like phenol, are harmful compounds which should be administered with caution to individuals and only in minute amounts. Other substances present in allergy shot preparations include alum (aluminium hydroxide), bacterial products, oil or lipid preparations, etc. Thus, not only the administration of the allergen itself may cause side effects but also the solvent, adjuvants, preservatives or other components of the injectible solution for use in immunotherapy. The injections of the diluted extracts of the allergen are administered on a regular schedule, usually twice a week or weekly at first, in increasing doses until a maintenance dose has been reached. This maintenance dosage reached after about 20 weeks and after about at least 20 allergy shots is then injected in the second treatment phase every 2 to 4 weeks for a period of 2 to 5 years. In case no benefit is achieved after 18 months of therapy the regimen is terminated.

During the desensitisation phase often moderate and sometimes severe side effects ranging from soreness and local swelling (wheal) or rash (flare) are caused. Moreover, systemic allergic effects may develop, such as generalized skin rash or hives and asthma or even anaphylaxis like anaphylactic shock may occur. Furthermore, other typical side effects occurring after injection include decrease of blood pressure, general itching and red eyes. Anaphylactic shock or anaphylaxis refer to an allergic reaction characterized by a sharp drop in blood pressure, hives or welts, and breathing difficulties, that occurs immediately, progresses rapidly and is life-threatening. Anaphylactic shock is the most severe reaction that can result from standard immunotherapy.

Due to the length of the current desensitisation therapy and the accompanying side effects, as described above, the dropout rate of patients during desensitisation therapy is very high. This further lowers the success rate of the therapy and increases the overall costs of therapy.

### Mechanism of desensitisation therapy

Typically, an allergic response is mediated by the binding of the allergen to receptor-bound IgE-antibodies at the cell surface of mast cells. The IgE-antigen interaction cross-links the receptor binding the IgE molecules resulting in activation of the mast cells. Activated mast cells induce a cascade of reactions involving the secretion of the contents of granulas e.g. the release of histamines, leukotrienes, etc.

These mediators among others result in vasodilation and contradiction of the smooth muscles. Thus, a drop of blood pressure in the blood vessels of the airways can occur which eventually results in an anaphylactic shock of the individual which may be fatal. In addition, various cytokines, especially proinflammatory cytokines, chemokines and other mediators are also released by, for example, activated mast cells, which mediators among others activate, or mediate the influx of other immune cells into the effected tissue.

Successful immunotherapy usually is accompanied by an increase in allergen specific IgG-antibodies and a decrease in allergen specific IgE-antibodies which normally occurs later. The precise mechanisms by which allergen immunotherapy achieves clinical improvements in the symptoms of allergic patients are still not completely clear, but it seems that IgG antibodies competing with membrane-bound IgE for binding to the allergen may prevent allergen-mediated IgE-receptor crosslinking and thereby protect against allergic reactions.

As a consequence one goal of desensitisation of allergic patients is to reduce or cut the IgE production, IgE-allergen interaction/binding, allergen-mediated immune cell activation and, preferably, redirect the immune response of the allergic patient towards an increased production of IgG antibodies, which, upon natural exposure to an allergen, compete with the IgE antibodies for binding to such allergen. Thus, an increased allergen specific IgG production reflects the efficacy of the desensitisation and seems to protect against allergic reaction.
The efficacy of desensitisation among others is reflected by:
- a drop in allergen-specific serum IgE titers, and/or
- a rise in allergen-specific serum IgG titers and/or
- a shift in the IgE /IgG ratio towards IgG, and/or
- a drop in mast and other immune cell activation, and/or
- a drop in concentrations of mediators such as histamine, leukotriene, tosyl-L-arginine methyl esterase (TAME), prostaglandine G2, pro-inflammatory cytokines, etc., and/or
- a drop in the infiltration of inflammatory cells, such as eosinophils, and/or
- a rise in the allergen tolerance of the allergic patient without signs, or with reduced signs of allergy (allergen dose, time-course, etc.), and/or
reduced allergic reactions of the allergic patient to environmental allergen exposure

### Medicaments for desensitisation therapy or allergen immunotherapy

Typically, an extract of the allergen containing composition, e.g. whole extracts of pollen are administered to the individual during desensitisation therapy. Seldom, isolated proteins or peptides containing the allergenic determinant are used in immunotherapy for desensitisation of individuals. However, injection of whole extracts of allergy inducing compositions like pollen, venom, etc. or recombinant or purified proteins or peptides suffer from ineffective delivery of said components to the cells and compartments to be treated and, as a consequence, often result in no or in an ineffective alleviation of the allergic response. Hence, the efficacy of desensitisation is very poor. In fact, since the uptake of the allergen components through the relevant cells is a prerequisite for effective stimulation of a protective antigen-specific immune response the delivery of the molecules containing the allergenic determinant to the relevant cells, in particular the antigen presenting cells (Apc) is of importance. In view of the poor uptake of allergen components by antigen presenting cells, high dosages of the allergen components are necessary for successful desensitisation..

Due to the insufficient or ineffective uptake of the molecules used for desensitisation, the molecules used for desensitisation may elicit local or systemic allergic reactions, as the molecules used for desensitisation are identical or very similar to the natural allergens causing the allergy. In this case for example mast cells are activated by the molecules used for desensitisation, resulting in the release of for example histamine which in the worst case can result in a life-threatening anaphylactic shock. With the desensitisation methods used to date this indeed happens sometimes (J Allergy Clin Immunol, 2004, 116:1129-36) and therefore common desensitisation protocols start with very small allergen doses, which are gradually increased to maintenance doses.

### Safety and efficacy of desensitisation therapy

A recent survey sent to all American Academy of Allergy, Asthma and Immunology physicians revealed, that more that 40 % of them reported near-fatal reactions towards desensitisation performed in their local practice and actually 41 fatal reactions were directly or indirectly reported (J Allergy Clin Immunol, 2004, 116:1129-36). In addition, the systemic administration of allergens usually leads to minor or moderate side effects not only at the site of injection but sometimes also systemically. Thus, under safety aspects a systemic administration of molecules for desensitisation is very risky in view of the frequency of incidence of side effects and at least is not favourably for the allergic patient due to unpleasant side effects such as local reactions of the skin, e.g. skin irritations, itching, skin swellings, etc.

The combined analysis of 75 trials involving immunotherapy showed, that overall there was a significant reduction in asthma symptoms and medication and an improvement in bronchial hyper-reactivity following immunotherapy (The Cochrane Database of Systematic Reviews, 2005, Issue 4, ISSN 1464-780X). Treatment of 4 individuals would avoid one deterioration in asthma symptoms, and treatment of 5 individuals would avoid one requiring increased medication.

To overcome the still remaining problems of conventional immunotherapy WO 02/028429 teaches methods of intranodal injections of extracts of whole allergen containing compositions, like pollen, venom, etc., or of recombinant allergy inducing protein allergens. Although the described methods allow for a significant reduction of allergy shots necessary for desensitisation, still the safety and efficacy of this system needs improvement. In particular, the amount of IgE antibodies and mast cell activation is still remarkable, thus, the risk of side effects is yet present. However, although a reduction of the amount of allergen to be injected to the individual is achieved, said amount must be of a certain level to attain the necessary level of efficacy. In addition there is always the risk of unintentionally injecting the allergens not into the lymph nodes, but into adjacent tissue, or even into blood vessels adjacent to the lymph node, resulting in an unintentional systemic antigen injection.

As discussed above, the methods and compositions presently available do not allow an efficient, low cost and safe desensitisation of individuals to allergens or to protein or peptide structures containing an allergenic determinant or epitope against which said individuals mount an allergic response.

### Object of the present invention

In view of the above, the object of the present invention is the provision of medicaments and methods allowing desensitisation of allergic patients with an increase in efficacy and safety of the treatment or desensitisation regime in a shorter time schedule.

The invention provides methods for desensitisation of individuals suffering from allergy. These methods use molecules with an allergen element comprising a peptide or protein structure with an allergenic determinant, which allergenic determinant is present in an allergen and which allergenic determinant is at least partially responsible for or at least partly causes the allergy in the individual. The allergen element is coupled covalently or non covalently to a so called "transduction element", namely a structure mediating the transport of the molecule from the extracellular into the intracellular space, i.e. the transduction element eases the transport of the molecule across membranes, in particular across cell membranes. These molecules are applied directly into the lymphatic tissue, for example injected into a lymph node, to elicit desensitisation of an allergic patient towards the allergen present in said molecule in the form of at least one protein or peptide structure comprising an allergenic determinant of said allergen. Subsequently these molecules are termed Intralyphatic-Transduction-AIIerGen (ITAG) -molecules.

### Advantages of the present invention over intranodal injection of pure allergen as described in WO 02/028429

Application of ITAG-molecules into lymphatic tissue, especially intranodal injection of ITAG-molecules, has significant advantages over the prior art, namely over the novel intranodal injection of allergens as described in WO 02/028429:
a) less adverse side effects, i.e. higher safety;
b) increased efficacy as compared to application of non-ITAG-molecules.

### Point a:

Less adverse side effects, i.e. increased safety is achieved since the transduction-element of ITAG-molecules results in rapid uptake of the ITAG-molecule by cells located adjacent to the site of the application/injection. Even if the ITAG-molecule accidentally is injected not into the lymph node but into an adjacent blood vessel, the ITAG-molecules will not be available systemically (which bears the risk of systemic allergic reactions), as they are rapidly taken up by, for example, blood cells or endothelial cells of the blood vessels. This prevents activation of mast cells by ITAG-molecules, as the vast majority of the ITAG-molecules do not reach mast cells. Even if an ITAG-molecule reaches a mast cell there are two competing reactions: uptake of the ITAG-molecule into the intracellular space of the mast cell, which most likely will not activate the mast cell, and binding of the ITAG-molecule by receptor-bound IgE at the cell surface of the mast cell, which will activate the mast cell and result in adverse side effects.

Due to reduced allergen doses in combination with the less frequent allergy shots which is a characteristic of intranodal injection necessary to achieve desensitisation, the allergic patient will also be subjected to smaller amounts of the non-allergen ingredients present in the allergy shots. Examples of such ingredients are adjuvants, stabilizers, solvents, filling substances, preservatives, etc. some of which such as phenol, alum, etc. are responsible for part of the adverse side effects of allergy shots, which part is reduced with the injected volume in combination with a reduced frequency of allergy shots.

### Point b:

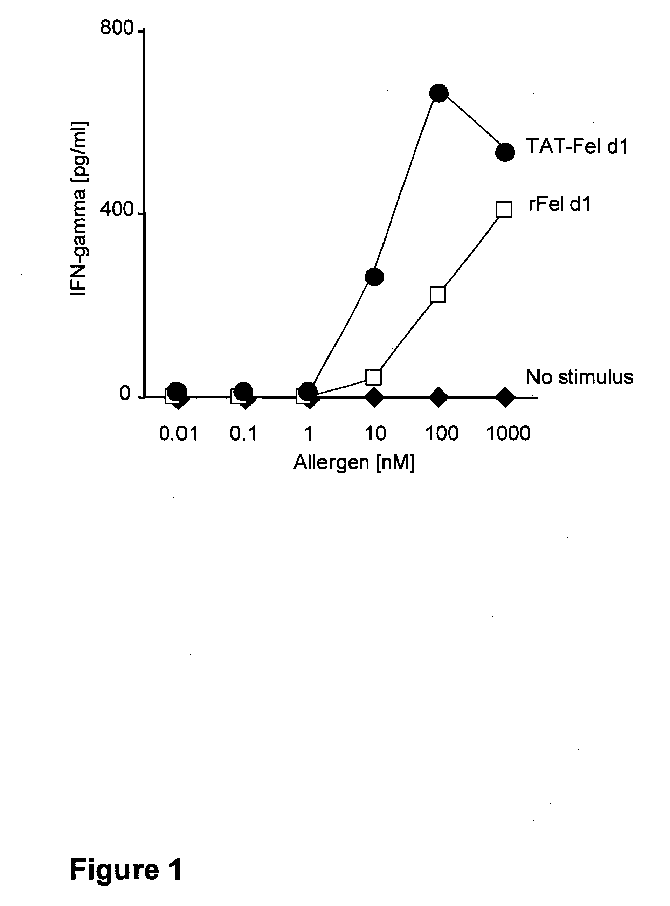

The increased efficacy of the ITAG-molecules compared to unmodified allergen is especially true since ITAG-molecules facilitate a more effective presentation of the allergenic determinants to the immune system and therefore result in a stronger protective immune response as compared to other allergens (see figures 1 to 4).

### Advantages of the present invention over subcutaneous injection

The application of ITAG-molecules directly into lymphatic tissue has several advantages over the prior art, namely over conventional subcutaneous injection of allergens:
1. lower antigen/allergen/vaccine doses is sufficient
2. less frequent injections in shorter time intervals result in desensitisation
3. less adverse side effects.

### Point 1

By direct application to the lymphatic tissue, for example by intranodal injection, the allergenic determinant is delivered closer to the site of immune response and the transduction element of ITAG-molecules results in more efficient transport of the protein or peptide structure containing an allergenic determinant into the cells involved in eliciting an immune response, resulting in a stronger immune response with lower doses of antigen/allergen/vaccine.

### Point 2

Point 2 is especially true as direct application to lymphatic tissue, for example by intranodal injections, results in desensitisation already after 3 injections within 28 days, whereas subcutaneous injections usually require about 30 to 80 injections over a period of 2 to 5 years

### Point 3

Less adverse side effects, i.e. increased safety is achieved since the transduction-element of ITAG-molecules results in rapid uptake of the ITAG-molecule by cells located adjacent to the site of the application/injection. Even if the ITAG-molecule accidentally is injected not into the lymphatic tissue, like the lymph node but into an adjacent blood vessel, the ITAG-molecules will not be available systemically (which bears the risk of systemic allergic reactions), as they are rapidly taken up by, for example, blood cells or endothelial cells of the blood vessels. This prevents activation of mast cells by ITAG-molecules, as the vast majority of the ITAG-molecules does not reach mast cells. Even if an ITAG-molecule reaches a mast cell there are two competing reactions: uptake of the ITAG-molecule into the intracellular space of the mast cell, which most likely will not activate the mast cell, and binding of the ITAG-molecule by receptor-bound IgE at the cell surface of the mast cell, which will activate the mast cell and result in adverse side effects.

Due to reduced allergen doses in combination with the less frequent allergy shots which is a characteristic of intranodal injection necessary to achieve desensitisation, the allergic patient will also be subjected to smaller amounts of the non-allergen ingredients present in the allergy shots. Examples of such ingredients are adjuvants, stabilizers, solvents, filling substances, preservatives, etc. some of which such as phenol, alum, etc. are responsible for part of the adverse side effects of allergy shots, which part is reduced with the injected volume in combination with a reduced frequency of allergy shots.

### Summary of the invention

It is one object of the present invention to provide an effective and safe method of delivery of allergenic determinants in an individual for desensitisation therapy.

It is a further object of the invention to provide specific compounds useful in medicaments for direct injection into intralymphatic tissue for desensitisation of individuals to allergens against which said individuals mounts an allergic response.

It is another object of the invention to provide specific compounds useful for desensitisation of individuals to allergens against which said individuals mounts an allergic response which are on the one hand safe, i.e. display reduced adverse side effects, and on the other hand, are efficient in eliciting a strong IgG based immune response in said individuals.

That is, the compounds described in the present application allow for a reduction of the number of injections and the amount of the compound to be injected while modulating the allergic condition efficiently with reduced side effects.

The invention relates to the use of recombinant, synthetic, or purified ITAG-molecules, comprising at least one protein or peptide structure containing an allergenic determinant, and at least one transduction element able to translocate the molecule across membranes, particularly across the cell membrane from the extracellular space into the interior of a cell in the manufacture of a medicament adapted for intralymphatic injection for desensitizing individuals to said allergen against which the individuals mount an allergic response.

In one aspect, the medicament is adapted for intranodal injection.

In other aspects of the invention, the medicament additionally contains suitable adjuvants, diluents, carrier, etc. for improved delivery of the above molecule comprising at least one protein or peptide structure containing an allergenic determinant, and at least one transduction element able to translocate the molecule from the extracellular space into the interior of a cell.

Furthermore, the invention provides kits for the desensitisation of allergy sufferers comprising the specific molecules as identified herein.

In addition, the present invention contemplates a method of modulating an allergic response in an individual comprising delivery by direct injection of a recombinant, synthetic, or purified ITAG-molecule comprising at least a protein or peptide structure containing an allergenic determinant, and at least one transduction element able to translocate the molecule from the extracellular space into the interior of a cell, into the intralymphatic tissue of said individual.

### Brief description of the drawings

Figure 1:
   PBMCs isolated from fresh blood samples of a patient allergic to cat dander (Fel d 1) were stimulated with the indicated concentrations of the following recombinant allergens: TAT-Fel d 1 (a ITAG-molecule comprising an N-terminal His-tag, followed by the HIV-tat sequence, followed by chain 2 of Fel d 1, followed by chain 1 of Feld 1), rFel d 1 (same as TAT-Fel d 1, except that the HIV-tat sequence is missing), and control buffer without any Fel d 1. After 45 min. the cell culture supernatant was harvested and the concentration of Interferon gamma (INF-gamma) determined by a commercial ELISA kit. High INF-gamma secretion induces a protective IgG immune reaction to the allergen used for desensitisation.
Figure 2:
   The same kind of supernatant of in vitro Fel d 1-stimulated PBMCs, isolated from cat dander allergic patients, was tested for Interleukin-10 (IL-10) using commercial ELISA kits. A high IL-10 secretion induces an allergen-specific tolerance of peripheral T cells.
Figure 3:
   Leukocytes prepared from fresh blood samples of cat dander allergic patients were subjected to an in vitro CAST® test, which determines the capacity of allergen-induced (in this case Fel d 1-induced) leukotriene release. Leukotriene-release exemplifies unwanted side effects of allergy shots used for desensitisation of allergy patients.
Figure 4:
   Mice were desensitized with TAT-PLA2, PLA2 purified from bee venom or control buffer by intranodal injection (3 injections, one every 2 weeks).The plasma IgG2 titers were determined after 6 weeks. High IgG2a titers indicate an allergy-protective immune response to the allergen used for desensitisation.

### Detailed description of the invention

### ITAG-molecule

An ITAG (Intralymphatic-Transduction-AllerGen) -molecule comprises at least two elements, a transduction element and an antigen or allergen element comprising a peptide or protein structure containing an allergenic determinant, wherein the
- Transduction element comprises an structure, which mediates the transport of the ITAG-molecule across the cell membrane, e.g. from the extra-cellular into the intra-cellular space
- Allergen element comprises at least one peptide or protein structure containing an allergenic determinant (allergy-mediating epitope) of an allergen

The ITAG-molecule in its entirety is a non-naturally occurring (artificial) molecule which may be obtained recombinantly, synthetically or by isolation and subsequent joining of the at least one transduction element and the at least one allergen element by covalent or non-covalent linkage.

### Transduction element and transduction sequences

The terms transduction sequence and transduction element are used in the text of the present application side by side as equivalent and having the same meaning. The term transduction element relates to structures, in particular to structures composed of amino acid residues, i.e. peptides or proteins which are able to translocate the molecule to which they are connected to across membranes, in particular cell membranes, like from the extracellular space into the interior of a cell, thus, passing the cell membrane.

The invention includes the use of various transduction elements for preparing ITAG-molecules. ITAG-molecules consist of at least one transduction element and at least one allergen element. The allergen element is at least one peptide or protein structure comprising an allergenic determinant. In general, all transduction sequences which are currently known and will be known in future are suitable to be used for the purposes of the present invention. Numerous suitable transduction sequences are described in the literature. These transduction sequences include viral sequences, homeoprotein sequences, leucine zipper sequences, arginine- and lysine-rich sequences, and various other sequences of proteins which are secreted despite the absence of a secretion signal sequence, etc.

In addition, numerous amino acid sequences, especially derived from viruses, e.g. HIV-tat or the protein VP22 derived from herpes simplex virus, promoting the transport of proteins, peptides and other classes of substances, such as, for example, nucleic acids or pharmaceutically active substances, into the interior of cells are known from the literature. For this purpose, these so-called transduction sequences are linked to the molecules to be transported (also called cargo molecules) either via covalent or via noncovalent linkages. Extracellular addition of the resulting compounds of transduction sequence and cargo molecule to cells is thus possible. The transduction sequence then brings about entry of the cargo molecule into the interior of the cell. This principle has likewise been described in numerous studies, especially for the HIV-tat sequence (US 5,804,604, Curr Opin Cell Biol, 2000, 12:400-6; J Biol Chem, 2001, 276:5836-40). Transduction elements represent not only naturally occurring transduction peptide sequences such as, for example, HIV-tat, VP22, Antennapedia, etc. but also, for example, peptidomimetics or other structures which are able to undertake the same function as the naturally occurring transduction peptide sequences.

### Functional fragment of a transduction element

In addition the invention also comprises ITAG-molecules comprising at least one functional fragment of a transduction element. With "at least one functional fragment of a transduction element" according to the invention are meant fragments of a transduction element such as the HIV-TAT molecule, as long as these fragments at least partially perform the same transduction process of the, for example, ITAG-molecule, as would the complete transduction element do. That means that a functional fragment of a transduction element still has the capacity to transport, for example, an ITAG-molecule from the extracellular to the intracellular space of a cell.

"At least one functional fragment of a transduction element" also comprises that there may be present more than one functional fragment of a transduction element within, for example, a single ITAG-molecule. Said functional fragments may origin from the same or from two or more different transduction elements.

It is also possible, that several copies of the same or of different transduction elements or fragments of transduction elements are present, for example, in a single ITAG-molecule.

### Inverted sequences

The term "inverted sequences" according to the invention means that the order in which the amino acid residues are arranged within a peptide or protein is inverted. As an example the inverted sequence of the amino acid sequence N-terminus-Alanin-Glycin-Prolin-Glutamin-C-terminus represents N-terminus-Glutamin-Prolin-Glycin-Alanin-C-terminus. It has been shown in the literature, that for example the HIV-tat sequence preserves it's transduction-activity if the amino acid sequence is inverted Proc Natl Acad Sci U S A, 1997, 97:13003-8).

### Allergen

An "allergen" according to the invention can be any molecule or portion thereof that elicits an allergic response. Common compositions containing allergens include bee venom, wasp venom, fire ant venom, pollens, including grass, tree and herb pollens, penicillin and other drugs, anesthetics, serum, animals, animal dander, cockroach or dust mite excrements, food allergens such as those found in peanuts, tree nuts, milk, fish, shellfish, eggs, soy, wheat, honey, fruits, allergens from viruses, bacteria, molds, protozoa, or allergens from plants such as latex. Likewise, an allergen containing composition may be a mixture of substances or a crude or purified extract of a generally allergenic composition which contain at least one molecule, like a protein, containing an allergenic determinant. Typically, the allergen comprises at least one protein or peptide structure containing an allergenic determinant. Common allergens are exemplied below.

### Allergen element, peptide or protein structure containing an allergenic determinant

The terms "allergen element" and "peptide or protein structure containing an allergenic determinant" are used in the text of the present application side by side as equivalent and having the same meaning. The term allergen element means any structure which can cause an allergic reaction.

An allergic determinant is any structure which is suitable for use in desensitisation therapy of allergic patients. Said structure can originate from any species different to said individual, or can originate from the same species as the species of said individual, or even can originate from said individual itself (e.g. in an auto-immune reaction). A peptide or protein containing an allergenic determinant is an amino acid sequence comprising at least 6 amino acids, preferably at least 8, preferably at least 10, preferably at least 15, preferably at least 20, preferably at least 50 amino acid residues, and said peptide or protein comprises at least one structure which represents said allergenic determinant. Said peptide or protein comprising an allergenic determinant can comprise one or can comprise more than one epitope, preferably up to 2, preferably up to 3, preferably up to 5 epitopes, but in addition to the allergenic determinant of the allergen can comprise further epitopes not involved in the allergic reactions, as an average protein usually contains more than 5 epitopes but only a minor portion of all epitopes are involved in or cause an allergic response.

Said allergic determinant can comprise only amino acid residues, or may additionally contain or may comprise only modifications such as posttranslational modifications of the amino acid residues, chemical modifications of the amino acid residues, enzymatic modifications of the amino acid residues and modifications of the amino acid residues due to other mechanisms, or can comprise combinations of amino acid residues and modifications of amino acid residues. Said allergic determinant can be recognized by any kind of antibodies such as soluble IgE, membrane-bound IgE, soluble IgG antibodies, etc. The allergic determinant can be present only in native or only in denatured peptides or proteins or can be present in native and denatured peptides or proteins and the allergic determinant can originate from material found in nature or from material not found in nature. Preferably said allergenic determinant is a peptide or protein derived from any species, especially from plant pollen, birch pollen, animal dander, cant dander, house dust, dust mite or cockroach excrements, plant or insect venom, bee, wasp or hornet poison, parts of plants or plant extracts or food ingredients, such as latex, poison of poison ivy, nuts, fruits, etc. or any other known allergen containing compositions such as the allergens listed below in this application or from an allergen so far not known to be an allergen.

The peptide or protein structure containing an allergenic determinant can be recovered from a natural source or can be a synthetic or non-naturally occurring substance such as a recombinant peptide, a peptide manufactured by biological or enzymatic synthesis for example by in vitro translation, or chemically or by other methods synthesized peptide, for example by using the Merrifield peptide synthesis, a peptide purified or recovered from natural sources or a mimetic chemical (including a peptide) or a peptidomimetic structure that elicits an allergic response similar to, or identical to a naturally occurring peptide or protein structure containing said allergenic determinant.

In case the peptide or protein containing said allergenic determinant is not isolated and/or found in nature, the amino acid sequence of said peptide or protein can be a sequence present in nature or it can be a sequence different from sequences present in nature, as long as said allergenic determinant present in said peptide or protein elicits an allergic response similar to, or identical to a naturally occurring peptide or protein structure containing said allergenic determinant. This is usually the case, if the structure of said allergenic determinant present in a sequence present in nature is very similar to the structure of the allergenic determinant present in a sequence not present in nature. Regarding peptide or protein sequences this is very often the case, if conservative amino acid exchanges distinguish the natural sequence form the non-natural sequence, e.g. chemical and/or physical similar amino acid residues are exchanged for each other. Such chemical or physical similar amino acid residues for example can be grouped according to their charge status (positive: R, H, K; negative: D, E; uncharged: A, N, C, Q, G, I, L, M, F, P, S, T, W, Y, V), or according to their volume and polarity (special: C; neutral and small: A, G, P, S, T; polar and relatively small: N, D, Q, E; polar and relatively large: R, H, K; nonpolar and relatively small: I, L, M, V; and nonpolar and relatively large: F, W, Y). Amino acid exchanges within a group are regarded as conservative exchanges (Mol Biol Evolution, 2002, .19:1022-1025). Very often antibodies binding to a naturally occurring peptide or protein structure also bind to a non-naturally occurring protein structure (are cross-reactive to the non-naturally occurring protein structure), if the difference between both structures are due to conservative amino acid exchanges.

The peptide or protein containing an allergenic determinant can comprise no chemical or posttranslational or other modifications, or can comprise chemical or posttranslational or other modifications found in nature or can comprise chemical or posttranslational or other modifications not found in nature, as long as said allergenic determinant present in said peptide or protein elicits an allergic response similar to, or identical to a naturally occurring allergen containing said peptide or protein structure containing an allergenic determinant.

### Adapted for intralymphatic or intranodal injection

The terms "adapted for intralymphatic injection" or "adapted for intranodal injection" or "adapted for direct injection into the axillary and/or inguinal lymph node" according to the invention means, that the composition, e.g. the substance, the peptide, protein, medicament, pharmaceutical composition, etc. adapted for intralymphatic or intranodal injection has physical, chemical, biological and other characteristics necessary or beneficial for injecting it as a medical treatment into lymphatic tissue of an individual, especially a human, even more preferably a human patient suffering from allergy and conditions associated with allergy. Such necessary or beneficial characteristics among others are: a sterile status of the composition, a viscosity allowing easy injection of the composition, osmolarity and pH-values permitting injection into lymphatic tissue without damaging cells and tissues within the lymphatic tissue. Furthermore compositions "adapted for intralymphatic injection" or "adapted for intranodal injection" according to the invention contain concentrations of all constituents of the composition allowing the application of appropriate amounts of all constituents in an appropriate volume into lymphatic tissue, preferably a volume of up to 0.01, preferably up to 0.05, preferably up to 0.1, preferably up to 0.2, preferably up to 0.3, preferably up to 0.4, preferably up to 0.6, preferably up to 0.8, preferably up to 1.0, preferably up to 2 mL. Furthermore compositions "adapted for intralymphatic or intranodal injection" should contain no or only limited amounts of potential harmful substances such as solvents and adjuvants, which might damage lymphatic tissue, if applied in too large quantities. With damage of lymphatic tissue is meant direct damage due to toxic effects to cells, due to chemical destruction of cells, due to indirect damage to cells for example by inducing inflammatory reactions, necrosis, etc. Furthermore a composition "adapted for intralymphatic or intranodal injection" according to the invention ideally comprises a protein or peptide structure comprising an allergenic determinant within an ITAG-molecule, which increases the efficiency of the transfer of the peptide or protein structure comprising an allergenic determinant to the cells within the lymphatic tissue, especially to cells responsible for mounting a anti-allergic immune response, e.g. an immune response which results in relief or disappearance of symptoms and/or disorders and/or disturbances caused by allergy.

Furthermore a composition "adapted for intralymphatic or intranodal injection" according to the invention ideally has some kind of safety-mechanism, which prevents accidental, systemic application of the composition, in case the injection misses the lymphatic tissue and in the worst case results in direct injection of the composition into the blood circulation. Such a safety-mechanism is a short extracellular half-life of the biological active substances, for example due to rapid transduction of the peptide or protein structure containing an allergenic determinant into cells, which, for example, prevents that the peptide or protein structure containing an allergenic determinant reaches and activates mast cells. ITAG-molecules, due to their transduction elements, comprise such a safety-mechanism.

### Other routes of administration

ITAG-molecules according to the present invention may also be administered by conventional routes, like subcutaneous administration or sublingual administration. In particular, ITAG-molecules are suitable for mucosal and systemic administration. That is, the ITAG-molecules may be administered orally, subcutaneously, transcutaneously (topical vaccination), intradermally, intramedullary, intrathecal, intraventricular, intranasally, conjunctival, intrabronchial, transdermally, intrarectally, intraperitoneally, intramuscularly, intrapulmonary, vaginally, sublingually, rectally, or intraocularly.

Thus, the medicament may alternatively be adapted for various administration ways. Preferred is an intra-NALT (nasal associated lymphoid tissue), aerosolized, sublingual or subcutaneous administration. Due to the presence of the transduction element, the risk of adverse side effects is reduced. In particular, the risk of activation and stimulation of mast cells or basophils which in turn release various mediators responsible for the allergic response of allergic patients is decreased since the molecules allow an improved transport of the allergen element into the cells. Consequently, the molecules stay in the area of administration and do not drain of into the blood system.

Hence, the ITAG-molecules as described herein are also suitable as medicaments for desensitisation of individuals suffering from allergy whereby the medicaments are administered via conventional routes, like subcutaneous, topical or sublingual administration.

Furthermore, it is possible to administer the ITAG-molecules adjacent to the lymphatic tissue, e.g. adjacent to the lymph node to achieve desensitisation of the individuals. Also adjacent administration may be sufficient due to the characteristics of the ITAG-molecules.

The ITAGmolecules according to the present invention comprise inter alia a peptide or protein structure containing said allergenic determinant. The ITAG-molecules present in the medicament, e.g. the desensitizing agent, may contain peptide or protein of a single species of structures, e.g. all structures are identical, for example a recombinantly, chemically or with other methods produced peptide or protein. In addition, the ITAG-molecules may comprise molecules wherein the peptide or protein structures have slight differences among each other, for example in chemical or posttranslational or other modifications, for example a peptide structure with glycosylation mixed with the same peptide structure with slightly different glycosylation or with partial glycosylation mixed with the same peptide structure without any glycosylation, or a complete peptide structure mixed with the same peptide structure without an N-terminal methionine mixed with the same peptide structure without its signal sequence, mixed with the same peptide structure with a C-terminal peptide sequence removed due to proteolysis, mixed with the same peptide structure originating from translation of a different allele of the same gene resulting in an amino acid sequence with slight differences, e.g. results in amino acid residue exchanged, deletions, or insertions, etc.

### Desensitisation

The present invention involves the delivery of an allergen by injection directly into a lymph node, or into other lymphatic tissues in order to modulate an allergic response of an individual (for example, to elicit an IgG response) more efficiently than is possible by subcutaneous injection. According to the invention, modulation of an allergic response includes, but is not limited to, diminution or elimination of response such as alterations in specific IgG levels, alterations in IgG ratios, alterations in specific IgE levels, lowered sensitivity to the allergen or to a cross-reactive allergenic agent, alterations in activated basophils (such as the reduction of the amount of surface/membrane/receptor-bound IgE), alterations in cytokine profiles (such as increase in type 1 cytokines e.g., IL-2 and IFN-gamma, vs. type 2 cytokines e.g. IL-4, IL-5), alterations in Radio-Allergosorbent Test (RAST) results, or skin tests, as well as diminution or elimination of symptoms of an allergic response, such as urticaria, itching, malaise, anxiety, angioedema, constriction of the chest, nausea, vomiting, diarrhea, abdominal pain, dizziness, dyspnea, wheezing, stridor, dysphagia, dysarthria, hoarseness, weakness, confusion, fall in blood pressure, collapse, loss of conseciousness, incontinence, cyanosis, mucus production, coughing, shock, stomach cramps, rhinitis, hay fever, asthma, inflammation, and the like.

### Intralymphatic in particular Intranodal administration of allergens

Intralymphatic or Intranodal administration of allergens has a number of advantages. Because lower doses of allergen can induce an IgG response more potently when injected directly into lymphatic tissue, in particular into a lymph node, there are fewer side effects than observed using the conventional allergy shot regime. Moreover, delivery of the allergen to the lymph node by injection is no more painful to the patient than regular subcutaneous injections. An additional advantage of this method is that only one to three treatments typically are necessary to desensitize an individual against an allergen. This lowers the risk of side effects or reaction to the administration, and results in significant cost savings compared to traditional allergy treatments.

Embodiments of the invention can be adapted to address any disease that includes an allergic-type response. For example, a number of newly discovered and/or little understood viral, bacterial, protozoal, and other diseases exist in the human population. To the extent that such diseases have a pathogenic component involving an allergic response or an IgE response, embodiments of the invention can be used to reduce the virulence of these diseases and/or to protect people against these diseases. The novel delivery method contemplated by this invention can also be used to alleviate the aspects of asthma and anaphylactic shock associated with hypersensitivity to allergens.

The molecules according to the present invention are preferably delivered in a physiologically acceptable carrier suitable for injection. In general, any physiologically acceptable carrier known for use with vaccines or allergy shots can be used in the practice of this invention. The choice of such carriers includes, without limitation, water, standard saline solutions, dextrose solutions and albumin water, and is easily within the skill of the art.

In one preferred embodiment, the ITAG-molecules of the present invention are delivered in combination with an adjuvant. The adjuvant may be, but is not limited to, one or more of the following: alum, BCG, aluminium hydroxide, aluminium phosphate, calcium phosphate, lipid emulsions, lipid or polymeric nano- or microspheres, micelles, liposomes, saponin, lipid A, or muramyl dipeptide, bacterial products, chemokines, cytokines and hormones, chitosan, starch, alginate, cellulose derivatives (e.g., ethyl cellulose, hydroxypropylmethyl cellulose), nucleic acids, or a nucleic acid construct. One or more of these components may be added to enhance or modify the immune response. Alternatively, the ITAG-molecule may be delivered without an adjuvant or in an aqueous form.

The ITAG-molecule may be delivered in a dose of about 0.05 µg to 50 µg and more preferably in a dose from about 0.1 µg to about 50 µg, like 0.1 µg to 10µg, although the optimal dose may vary depending on the allergen being injected, the weight of the patient, the immune system of the patient, and the like. Effective treatment in many cases may be accomplished with one delivery. In some embodiments, treatment includes from 1 to 15 injections. In preferred embodiments, treatment includes from 1 to 5 injections and more preferably 1 to 3 injections. For initial treatment injections may be delivered periodically, e.g. over a course of days, once or twice per month or year, or several times a year. For maintenance of immune response, injections may be delivered in intervals of several months to several years.

Optionally, a lymph node may be visualized during the injection procedure. Ultrasound, radiological, or other visualization means such as computerized axial tomography (CAT scan), can be used to visualize the lymph node and monitor location of the needle and changes in the lymph node, such as swelling. Injection into the axillary and inguinal lymph nodes is preferred due to ease of ultrasound guided location and injection.

During administration of the ITAG-molecule, the patient's vital signs typically are closely monitored, and the lymph node reaction is monitored, for example, by ultrasound or other visualization methods.

The technique used for injection is within the skill of the art. One method is to use a dual-chamber syringe in which the lyophilised ITAG-molecule is included in one chamber and a liquid carrier in the other, to be mixed prior to injection. In another method a single chamber syringe containing the allergen in liquid formulation may be used.

In preferred embodiments of the inventions, the ITAG-molecule is delivered directly to lymphatic tissue, preferably, a lymph node during both desensitisation treatment and maintenance treatment. Alternatively, the allergen may be delivered directly to lymphatic tissue, preferably, the lymph node during the desensitisation phase and subcutaneously during a maintenance phase. Some of the benefits of the invention may be conferred if intralymphatic therapy is employed during desensitisation and subcutaneous therapy is employed during maintenance. If maintenance therapy is done by subcutaneous allergy shots preferably not ITAG-molecules but conventional extracts, for example cat dander extract, or pollen, house dust or other extracts, or recombinant or purified molecules comprising an allergenic determinant but not comprising a transduction element are used.

The invention provides a method which makes it possible for allergen elements to be supplied in a very targeted manner to cells in order to achieve an efficient, specific immune reaction. The method makes it possible to convey allergen elements efficiently from the extracellular space to the intracellular space of the cell.

In the prior art it has been described frequently as being very disadvantageous that transduction sequences are not specific for particular types of cells, but are equally active in all types of cells (Proc Natl Acad Sci U S A, 2000, 97:13003-8). In contrast, in the present invention, an universal functionality of transduction elements is greatly desired and advantageous which has not been realized in the prior art to date. That is, it is preferred that the transduction element results in a transduction of at least 20 % of the cells surrounding the site of e.g. the intralymphatic application, and preferably of at least 30 %, for example 50 % of the cells which for example can be determined by use of fluorescently or otherwise labelled ITAG molecules, whose fate is monitored using microscopy, FACS analysis or other methods known in the art.

In another preferred embodiment the universal functionality of transduction elements is indicated by transduction of the ITAG molecules in at least 2 preferably at least 3 more preferably at least 4 different types of cells selected from the list comprising erythrocytes, leucocytes, thrombocytes, endothelial cells, myocytes, adipocytes, keratinocytes, fibrocytes, fibroblasts, hepatocytes and kidney cells. This can be monitored by using labelled ITAG molecules, whose fate is monitored using methods such as microscopy, FACS analysis or other methods known in the art.

In still another embodiment the universal functionality of transduction element is indicated by a rapid disappearance of ITAG molecules from the circulation as a result of the rapid transduction of the ITAG molecules into blood cells or into endothelial cells lining the blood vessels. This rapid disappearance results in less than 60 min., preferably in less than 30 min. in a reduction of the ITAG molecules below a threshold value, which is preferably as low as the concentration of the allergen concentration, which would cause in an in vitro CAST® assay not more than 5 % of the maximal leukotriene release.

This universal functionality results in improved safety of ITAG-molecules, as an accidental, systemic application of ITAG-molecules is unlikely to reach to a significant extent cells such as mast cells, which would promote unwanted allergic reactions such as histamine release. This is the case as only a minor fraction of all cells are mast cells and therefore e.g. ITAG-molecules accidentally injected intravenously would to a very large extend be transferred into the intracellular space of ubiquitous blood cells or endothelial cells lining the blood vessels. The best-known and characterized amino acid sequences which can be used as transduction element for the purposes of the invention are the HIV-tat and, the VP22 and the antennapedia sequences.

### Viral peptide sequences suitable as transduction elements

The peptide sequences suitable as transduction elements for the purposes of this invention include inter alia viral proteins or partial sequences of viral proteins such as, for example, the HIV transcriptional activator protein (HIV-tat). The suitable Tat proteins include besides the Tat protein of the HIV-1 virus also the Tat proteins of other lentiviruses (Proc Natl Acad Sci U S A, 2000, 97:13003-8). Numerous modified Tat peptides have been described as sequences able to bring about transduction. These include Tat peptides which represent only partial sequences of the Tat protein, Tat peptides which comprise point mutations, Tat peptides in which the sequence is reversed (inverted), or Tat peptides which comprise unusual amino acids such as, for example, D isomers of amino acids, etc. (Proc Natl Acad Sci U S A, 2000, 97:13003-8). All these variations of peptide sequences are therefore generally suitable as transduction elements. Peptides also suitable for the purposes of the present invention as transduction elements are those derived from other viruses such as, for example, VP22 (herpes simplex virus-1 VP22 tegument protein) (Proc Natl Acad Sci U S A, 2000, 97:13003-8). At present, commercial expression vectors comprising a VP22 sequence suitable for transduction are also available. These expression vectors therefore permit VP22 fusion proteins to be prepared (Voyager^{™} VP22 system, Invitrogen, Breda, the Netherlands). Other viruses, e.g. Marek's disease virus-1, a virus which causes lymphoma in chickens, also express a protein which is related to VP22 and which is likewise suitable as transduction element (J Gen Virol, 2000, 81:2219-30). These proteins and partial sequences of these proteins are mentioned only as examples, and numerous further peptides are known at present, and will become known in future, which are suitable as transduction elements for the purposes of the invention.

### Homeoproteins suitable as transduction elements

A further group of transduction elements suitable for the purposes of the present invention are peptides derived from the drosophila homeotic protein antennapedia (ANTp) (Proc Natl Acad Sci U S A, 2000, 97:13003-8). Among others, ANTp peptides suitable as transduction element are those comprising an inverted sequence of ANTp, comprising D isomers of amino acids, or comprising point mutations in their sequence (Curr Opin Cell Biol, 2000, 12:400-6). It is additionally expected that numerous further ANTp sequence modifications are also possible and will presumably make transduction possible. ANTp peptide variants are also referred to as transport peptides. Further homeoproteins such as, for example, engrailed 1 (En1), engrailed 2 (En2), Hoxa-5, Hoxc-8, Hoxb-4 and KNOTTED-1 (Curr Opin Cell Biol, 2000, 12:400-6) likewise comprise sequences which can be used as transduction element for the purposes of the present invention. KNOTTED-1 is in fact a plant protein, but is likewise suitable as transduction element in animal cells. These peptides are mentioned only as examples, and numerous further homeoproteins which comprise peptide sequences which may be suitable as transduction element for the purposes of the invention are known (Nucleic Acids Res, 2001, 29:291-3). Further previously undisclosed homeoproteins may also comprise sequences suitable as transduction element.

### Leucine zipper proteins suitable as transduction element

A further group of sequences suitable as transduction element for the purposes of the present invention are peptides comprising a leucine zipper domain. Examples of proteins whose leucine zipper domains can be used as transduction sequence are, for example, human cFos-(139-164), human cJun-(252-279), or the yeast transcription factor yeast GCN4-(231-252) (J Biol Chem, 2001, 276:5836-40). Further leucine zipper proteins already known or which will become known in future are likewise suitable as transduction element for the purposes of the invention.

### Arginine- or lysine-rich peptides suitable as transduction element

Arginine-rich peptides, frequently derived from RNA- and DNA-binding proteins, represent further peptide sequences which can be used as transduction elements for the purposes of the present invention. Examples of such sequences are HIV-1 rev-(34-50), flock house virus coat protein FHV coat-(35-49), BMV Gag-(7-25), HTLV-II Rex-(4-15), CCMV Gag-(7-25), P22 N-(14-30), lambda N-(1-22), phi 21 N-(12-29) and PRP6-(129-144) from yeast (J Biol Chem, 2001, 276:5836-5840)]. It is likewise possible to use for the purposes of the invention polyarginine peptides having 4 to 16 (J Biol Chem, 2001, 276:5836-40) or else having more than 16 arginine residues. In addition to polyarginine peptides, peptides which can also be used as transduction elements are those which, besides arginine, also comprise further amino acids, e.g. the W/R peptide (RRWRRWWRRWWRRWRR (Curr Opin Mol Ther, 2000, 2:162-167) or the R9-Tat peptide in which the 9 central amino acid residues of the total of 11 amino acids of a Tat peptide have been replaced by arginine residues (GRRRRRRRRRQ) (J Biol Chem, 2001, 276:5836-5840). It has additionally been possible to show that peptides which for example consist of nine lysine residues also have the ability to act as transduction element for the purposes of the invention (J Gen Virol, 2002, 83:1173-81). These peptides are mentioned only as examples and numerous further arginine- or lysine-rich peptides are suitable to be used as transduction element for the purposes of the invention (J Biol Chem, 2001, 276:5836-40, J Gen Virol, 2002, 83:1173-81). Further arginine- or lysine-rich peptides already known or to become known in future are presumably also suitable as transduction element. Sequences comprising guanidino or amidino groups are also suitable as transduction element for the purposes of the present invention (US patent US 2002009491 AA)..

### Proteins without signal sequence which are suitable as transduction element

A number of further proteins have the ability, without a secretion signal sequence being present, to penetrate the cell membrane from the inside to the outside, i.e. be secreted. These proteins are frequently also able conversely to penetrate into the interior of the cell from the outside. These proteins or partial sequences of these proteins can thus also be used as transduction elements for the purposes of the present invention. Some examples of such proteins are fibroblast growth factor 1 (FGF-1), fibroblast growth factor 2 (FGF-2), caveolin-1, lactoferrin, thioredoxin, interleukin 1 beta and ciliary neurotrophic factor (CNTF) (Curr Opin Cell Biol, 2000 12:400-6), or interleukin 1 alpha, vas deferens protein, platelet-derived endothelial cell growth factor (PR-ECGF), thymosin, para-thymosin, 14.5 kDa lectin (L14), transglutaminase, thioredoxin-like protein (ADF), sciatic nerve growth-promoting activity, factor Xllla, mammary-derived growth inhibitor, galectin, rhodanase (US patent US 6083706). These peptides are mentioned only as examples, and numerous further peptides are known or will become known in future which are suitable as transduction element for the purposes of the invention.

### Toxins suitable as transduction element

Many toxins or partial sequences of toxins have the property of acting as transduction element, such as, for example, following toxins: complete abrin, complete ricin, complete modeccin, complete pseudomonas exotoxin A, complete diphtheria toxin, complete pertussis toxin, complete Shiga toxin, the A chain of ricin, the A chain of abrin, the A chain of modeccin, the enzymatically active domain of pseudomonas exotoxin, the A chain of diphtheria toxin A, the enzymatically active domain of pertussis toxin, the enzymatically active domain of Shiga toxin, gelonin, pokeweed antiviral protein, saporin, tritin, barley toxin and snake venom peptides (EP 0359347). These toxins mentioned as examples, and many further other toxins not expressly mentioned or toxins to become known in future can be used as transduction element for the purposes of the present invention.

### Controlling the efficiency of transduction elements

The efficiency of transduction can be optimized by varying the length of, for example, a poly-arginine chain or by specific selection of, for example, only a partial sequence of the HIV-tat sequence, or by systematic exchanges of individual amino acid residues to optimize the function of the HIV-tat sequence so that very efficient transduction of the corresponding ITAG-molecule takes place. A very efficient transduction has the advantage that the efficacy of the ITAG-molecule is increased and/or that the necessary dose of ITAG-molecules can be reduced, in turn saving vaccine costs. A very efficient transduction of the corresponding ITAG-molecule in turn has also the advantage that possibly fewer side effects occur.

### Examples of minimal sequences acting as transduction element

It is not necessary for all the transduction sequences mentioned as examples to be in the form of the complete protein or peptide as constituent of the ITAG-molecule in order to be effective as transduction element for the ITAG-molecule for the purposes of the invention. On the contrary, a minimal sequence region which can be used as transduction sequence is known for many of said proteins or peptides. This sequence region includes for example for HIV-tat for example the following sequence: (Gly)-Tyr-Gly-Arg-Lys-Lys-Arg-Arg-Gln-Arg-Arg-Arg (seq.ID. No. 11), for VP22 the following sequence: Asp-Ala-Ala-Thr-Ala-Thr-Arg-Gly-Arg-Ser-Ala-Ala-Ser-Arg-Pro-Thr-Glu-Arg-Pro-Arg-Ala-Pro-Ala-Arg-Ser-Ala-Ser-Arg-Pro-Arg-Arg-Pro-Val-Glu (Seq.ID.No.12) and for antennapedia the following sequence: Arg-Gln-Ile-Lys-Ile-Trp-Phe-Gln-Asn-Arg-Arg-Met-LysTrp-Lys-Lys (Seq.ID.No. 13)(Trends Pharmacol Sci, 2000, 21:45-8). It is additionally possible for the sequences also to be used in the form of fragments which do not correspond to the currently known minimal functional sequence segments, as long as the resulting sequence is still functional for the purpose of the transduction of the ITAG-molecule.

Transduction elements need therefore not be in the form of the complete protein or the complete molecule as constituent of the ITAG-molecule in order to be effective as transduction element. Testing of the functionality of a transduction element can be ascertained for example by using fluorescence-labelled transduction elements or by using enzyme-labelled transduction elements or by using transduction elements labelled with metal particles. The transduction elements labelled in these ways are administered to an experimental animal or to cells cultivated in vitro, and the fate of the transduction elements is followed using methods such as FACS (fluorescence activated cell sorting), microscopy, confocal fluorescence microscopy, electron microscopy etc. These techniques for checking the functionality of transduction elements are described in the literature, and some of them have already been used to ascertain the functionality of sequences for transduction (J Biol Chem, 2000, 276:5836-40, Science, 1999, 285:1569-72).

Further, the term allergen element relates to a protein or peptide structure comprising allergenic determinant/allergenic determinants which are also known as epitope/s of allergens. Thus, the allergen element may be the whole allergen, e.g. the protein, or is a part of the allergen, i.e. a fragment thereof, like a peptide, encompassing at least one allergenic determinant or allergenic epitope. The at least one allergenic determinant or allergenic epitope is able to elicit an allergic response to the allergen from which said allergenic determinant originated. The allergenic determinant can comprise one or more than one amino acid residues or peptide or other structures capable to elicit an allergic response such as sugar structures, phosphorylated amino acids, lipids, etc. or combinations thereof. The allergenic determinant can be a continuous epitope (= not dependent on conformation = present in for example native and denatured proteins) or a discontinous epitope (= dependent on conformation = only present in native, folded, but not present in denatured proteins). Allergenic determinants according to the invention comprise preferably at least 6, at least 8, at least 10, at least 15, at least 20 amino acids residues or other structures suitable as epitope. It is possible to use not only proteins and peptides, but also carbohydrate structures, lipids, e.g. lipopolysaccharides, lipoteichoic acids and other constituents of bacterial membranes (CD1 b binds, for example, sugar structures and lipids), nucleic acids such as, for example, DNA comprising CpG motifs, organic substances such as, for example, latex or pharmaceutically active substances such as penicillin as source for allergenic determinants for the purposes of the invention. The allergenic determinant may be derived from humans, animals, plants, fungi, parasites, unicellular or multicellular microorganisms, viruses and other life forms. The allergenic determinant may have been isolated from biological material, have been prepared as recombinant allergens or have been prepared by synthesis, e.g. by peptide synthesis. Synthetically prepared allergenic determinants may be substances which occur in nature or which do not occur in nature but are obtainable by chemical synthesis. Examples of non-naturally occurring substances which are, however, suitable as allergenic determinants in some circumstances are, for example, synthetically prepared substances which are present in medicaments, or synthetic peptides having amino acid sequences which do not occur in nature, or peptidomimetics, etc. Naturally occurring or synthetic or recombinant allergenic determinants can be modified by molecular-biology, enzymatic, chemical and other methods in order to confer on them properties which are more advantageous for the particular application. These advantageous properties may be, inter alia, a higher or lower activity as allergen, a broader or a more specific action as allergen, a better solubility in hydrophilic or hydrophobic solvents, a greater permeability of the allergen elements for cell membranes, etc., a higher or lower half-life in vivo or in vitro, a lower toxicity, a better detectability of the allergen element in vivo or in vitro after application of the allergenic determinant in the form of an ITAG-molecule etc. It is additionally possible for the purposes of the invention to combine a plurality of allergenic determinants in an allergen element (J Neurosci Res, 1996, 46:258-62). For this it is possible for identical allergenic determinants to be present in more than one copy in the allergen element, or it is possible for example for different variants of the same allergenic determinant to be combined in an allergen element. Combination of allergenic determinants, e.g. of allergenic determinant 1, and other allergenic determinants, e.g. of allergenic determinant 2, in an allergen element is also possible, etc. Further combinations such as, for example, allergenic determinant 1 in more than one copy and allergenic determinant 2 in a single copy may also be combined in an allergen element, etc. It is additionally possible also for one or more different and/or one or more identical antigen elements to be present in a ITAG-molecule. It is possible in principle for all possible combinations of singly and multiply present identical or altered copies of allergenic determinants derived from one or more different allergens to be combined in one or more identical or different allergen elements within an ITAG-molecule for the purposes of the invention.

### Allergens which can be used as antigen element

Numerous allergens or fragments thereof comprising the peptide or protein structure comprising an allergenic determinant, have been described in the literature to date. The allergens or fragments thereof comprising the antigenic determinant or epitope specifically known hereinafter can be used as antigen element for the purposes of the invention. Further allergens and variants of allergens which can likewise be used as antigen element for the purposes of the invention are known in the art (Offical list of allergens, IUIS Allergen Nomenclature Subcommittee, ftp://biobase.dk/pub/whoiuis/aiiergen.iist, www.allergome.org). That means, the allergen element according to the present invention preferably comprises at least one allergenic determinant, e.g. an epitope, which is potentially capable to trigger an allergic response. Preferably the complete molecules or fragments thereof of the allergens Fel d 1, Bet v 1, Der p 1, Der p 2 and PLA2 are used according to the invention as an allergen element within a ITAG-molecule. An alternative name for PLA2 is API m 1. Furthermore certain allergens may show cross reactivity to each other, such as for example Bet v 1 and Api g1 (Eur J Biochem, 1995, 233:484-489). In addition, certain sources of allergens contain not only one major allergen almost exclusively responsible for the allergic reactions, but instead do contain small groups of preferably 2 to 10 different allergenic determinant-containing, allergy-promoting substances. These allergenic determinants can be combined for allergy-treatment in the form of individual ITAG-molecules, each ITAG-molecule comprising a different allergenic determinant as allergen element. Alternatively these allergenic determinants can be combined in one or several ITAG-molecules which ITAG-molecules each contain more than one allergenic determinant as an allergen element within the ITAG-molecule. Examples of such sources of multiple allergens among others are Phleum pratenses, Betula verrucosa, Dermatophagoides pteronissinus, Dermatophagoides farinae, Artemisia vulgaris, Aspergillus fumigatus, Arachis hypogaea, Alternaria alternate, Cladosporium herbarum and Apis mellifera (see for examples in the subsequent list of allergens for groups of allergens from the same species such as Phl p 1, Phl p 2, Phl p 3, and Phl p 5, or Bet v 1, Bet v 2, and Bet v1d, or Der p 1, Der p 2, Der p 5, and Der p 7, or Asp fl 1, Asp fl/a, Asp f 2, Asp f 3, Asp f 4, Asp f 6, and Asp f 8, or Ara h 1, and Ara h 2, or Hev b 1, Hev b 2, Hev b 3, Hev b 5, Hev b 6, Hev b 7, and Hev b 8, or, Api m 1, Api m 2, PLA2 (Api m 1), and Api m 4).

The following list of allergens are arranged according to groups such as allergens from plants and grasses, from trees, from mites, from fungi, from insects, from foods and from other allergens such as, for example, latex allergens. The listing in the enumerations is as follows: scientific name of the organism, a commonly used abbreviation of the allergen directly followed by the GeneBank accession No. of the allergen (written in parentheses), where known.

### Plant and grass allergens:

Ambrosia artemisiifolia, Amb a 1 and Amb a 2; Mercurialis annua, Mer a 1 (Y13271); Parietaria judaica, Par j 1 (X77414), Par j 2 (X95865; X95866); Cynodon dactylon, Cyn d 1 (S83343); Dactylis glomerata, Dac g 3 (U25343); Holcus lanatus, Hol I 1 (Z27084, Z68893); Lolium perenne, Lol p 1 (M57474), Lol p 2 (X73363) Lol p 5 (M59163); Phalaris aquatica, Pha a 1 (S80654); Phleum pratense, Phl p 1 (X78813), Phl p 2 (X75925), Phl p 3, Phl p 5 (X74735); Artemisia vulgaris, Art v 1 (Z48967)

### Tree allergens:

Alnus glutinosa, Aln g 1 (S50892); Betula verrucosa, Bet v 1 (X15877), Bet v 2, Bet v1d; Carpinus betulus, Car b 1 (X66932, X66918); Corylus avellana, Cor a 1 (X70999, X71000, X70997, X70998, Z72439, Z72440, AF136945, AF323973, AF323974, AF323975); Ligustrum vulgare, Lig v 1 (X77787, X77788); Olea europea, Ole e 1 (S75766), Ole e 9 (AF249675); Syringa vulgaris, Syr v 1 (X76541); Cryptomeria japonica, Cry j 1, Cry j 2 (D29772, D37765); Cupressus arizonica, Cup a 1 (AJ278498); Cupressus sempervirens, Cup s 1 (AF257491); Juniperus ashei, Jun a 2 (AJ404653)

### Mite allergens:

Blomia tropicalis, Blo t 5 (U59102); Dermatophagoides farinae, Der f 1, Der f 2, Der f 11; Dermatophagoides pteronyssinus, Der p 1, Der p 2, Der p 5, Der p 7; Lepidoglyphus destructor, Lep d 2 (X81399); P. americana, Cra-A; T. putrescentiae, Tyr p 2

### Animal allergens:

Bos domesticus, Bos d 2 (L42867); Equus caballus, Equ c 1 (U70823); Felis domesticus, Fel d 1 (M74952, M74953)

### Fungal allergens:

Alternaria alternata, Alt a 1 (U82633), Alt a 2 (U62442); Aspergillus flavus, Asp fl 1 (AF137272); Aspergillus fumigatus, Asp f 1 (M83781, S39330), Asp fl/a, Asp f 2 (U56938), Asp f 3 (U20722, U58050), Asp f 4, Asp f 6, Asp f 8; Aspergillus niger, Asp n 18; Aspergillus oryzae, Asp o 13 (X17561); C. comatus, Cop c 1; Penicillium chrysogenum, Pen ch 13 (AF193420), Pen ch 20 (S77837); Penicillium oxalicum, Pen o 18 (AAG44478); Malassezia sympodialis, Mal s 1 (X96486); Cladosporium herbarum, Enolase, Cla h1

### Insect allergens:

Apis mellifera, Api m 1 (X16709), Api m 2 (L10710), Api m 4 (X02007); PLA2 (X16709); Blattella germanica, Bla g 1 (AF072219, L47595, AF072221, AF072220), Bla g 2 (U28863), Bla g 4 (U40767), Bla g 5 (U92412); Periplaneta americana, Per a 1 (AF072222), Per a 3 (L40819); Dolichovespula maculata, Dol m 1 (X66869), Dol m 2 (L34548), Dol m 5 (J03601); Dolichovespula arenaria, Dol a 5 (M98859), Polistes annularies, Pol a 5 (M98857); Vespula vulgaris, Ves v 1 (L43561), Ves v 2 (L43562), Ves v 5 (M98858); Myrmecia pilosula, Myr p 1 (X70256), Myr p 2 (581785)

### Food allergens:

Salmo salar, Sal s 1 (X97824); Bos domesticus, Bos d 4 (M18780), Bos d 5 (X14712); Gallus domesticus, Gal d 1 (J00902), Gal d 2 (J00992); Metapenaeus ensis, Met s 1 (U08008); Hordeum vulgare, Hor v 15 (X63517); Oryza sativa, Ory s 1 (U31771); Apium graveolens, Api g 1 (Z48967); Daucus carota, Dau c 1 (U47087, D88388); Malus domestica, Mal d 1 (X83672); Pyrus communis, Pyr c 1 (AF057030); Persea americana, Pers a 1 (Z78202); Prunus armeniaca, Pru ar 1 (U93165); Prunus avium, Pru av 1 (U66076); Arachis hypogaea, Ara h 1 (L34402), Ara h 2 (L77197); Bertholletia excelsa, Ber e 1 (M17146); Juglans regia, Jug r 1 (U66866), Jug r 2 (AF066055); Ricinus communis, Ric c 1 (X54158); Sesamum indicum, Ses i 1 (AF240005); Apium graveolens, Api g 1 (Z48967)

### Further allergens (latex):

Hevea brasiliensis, Hev b 1 (X56535), Hev b 2, Hev b 3, Hev b 5 (U42640), Hev b 6 (M36986), Hev b 7, Hev b 8
These known allergens are mentioned merely by way of example, and further allergens which can likewise be used in antigen elements for the purposes of the invention are known in the art.

In a preferred embodiment, the antigen element comprises the Fel d 1 or fragments thereof. The allergen Fel d 1, i.e. the Fel d 1 antigen, is a protein composed of two chains, chain 1 and chain 2. In nature, these two chains are connected by disulfide bridges and form a heterodimer known as Fel d 1. In a particularly preferrerd embodiment, the antigen element comprises the Fel d 1 chains 2 and 1 being covalently linked to one another. The order of the two chains is not decisive, however, it is preferred that chain 2 is at the N-terminus of the antigen element and chain 1 is at the C-terminus, as this more accurately resembles the native conformation of Fel d 1 (2003, J Biol Chem, 2003, 278:40144-40151; WO 2004/094639 A2). Between these two covalently linked chains spacer elements may be present. Of course, the antigen element may contain only one of these two Fel d 1 chains or only fragments of one or both of these chains. The Fel d 1 antigen element may also comprise more than one copy of at least one fragment or epitope of Fel d 1 and/or the Fel d 1 antigen element may comprise modifications of the Fel d 1 sequence as described elsewhere in this patent application. The sequences of Fel d 1 are disclosed in US 5,328,991 which is incorporated herein by reference.

### Further elements which may be present in ITAG-molecules

Besides the two elements already described - transduction element, and antigen element - which must be present in an ITAG-molecule, it is also possible for further optional elements to be present in the ITAG-molecule. These optional elements include, for example, targeting elements which direct the ITAG-molecule, once it has been delivered to the intracellular space, to those organelles or intracellular regions, which are involved in processing and/or binding of antigens to MHC-molecules, and elements which make it possible to isolate or detect the ITAG-molecules. An example of a targeting element is the invariant chain of the MHC molecule. In general targeting elements mediate the intracellular transport of the ITAG-molecule to for example the endoplasmatic reticulum, the Golgi apparatus, the trans-Golgi network, endosomes and lysosomes but not the nucleus. Such targeting elements are known from the literature (Adv Immunol, 2000, 75:159-208; J Cell Biol, 1995, 131:351-69; J Biol Chem, 1998, 273:18966-73; J Cell Sci, 2002, 115:185-94; Int Immunol, 1994, 6:973-82).

Elements which make it possible to isolate or detect the molecules are referred to in the art as "tags" and in this patent application are therefore referred to as tag elements. Examples of tag elements are His-tags, FLAG-tags, Myc-tags, fluorescent protein tags, enzyme tags, radionuclides as tags, etc.

Further elements optionally present in the ITAG-molecules may be spacer elements, i.e. elements which are arranged between the other elements and whose task is to couple these elements covalently or non-covalently, for example by biotin-streptavidin-interaction, to one another. Examples of spacer elements are short peptides such as short amino acid sequences from 2 to 6 amino acids in length consisting of glycine and/or alanine and/or serine, crosslinkers, natural or synthetic polymers such as substituted or unsubstituted hydrocarbons, etc. Peptide spacer elements might comprise protease recognition sequences for example for thrombin, factor Xa, or enterokinase.

It is also possible that certain elements have the function of two or more elements. For example, many tag elements can be used both for isolation and for detection of the ITAG-molecule, or an allergen element present in a ITAC-molecule might also be used for detection and/or isolation of the ITAC-molecule if, for example, an antibody against the allergen element is available, etc.

### Structure of the ITAG-molecules

In general , any desired arrangement of the individual elements of the ITAG-molecule is possible. Each element may be present once or more than once in the ITAG-molecule. The minimum requirement is the need for the presence of at least one transduction element and at least one allergen element having a protein or peptide structure comprising at least one allergenic determinant. Additional elements such as tag elements, targeting elements, spacer elements, protease recognition sites, etc. may optionally be present but need not be present. If elements are present more than once, they may be present in the form of identical copies, or different elements may be present, in each individual case as a single copy or in more than one copy.

Especially two or more identical or different copies of allergen elements may be present in an ITAG-molecule, which allergen elements may comprise protein or peptide structures comprising allergenic determinants of allergens from the same and/or different allergens. This may serve for desensitisation of an individual reacting allergic to two or more allergens at the same time.

Two or more different transduction elements may be present in an ITAG-molecule.

For example, a Tat sequence and a VP22 sequence can serve to make transduction more efficient. It is also possible for example to use two or more tag elements in a ITAG-molecule, e.g. a His tag and a FLAG tag, in which case for example the His tag may be used to isolate the ITAG-molecule and for example the FLAG tag serves to detect the ITAG-molecule.

The position of the individual elements within the ITAG-molecule can also be varied, as long as at least one transduction element, and at least one allergen element is present. It is also possible for all or some of the elements of the ITAG-molecule to be present not in the form of a linear sequential arrangement of elements but as circular or as branched element structure or else in the form of dendrimers, or as a combination of linear and/or branched and/or circular and/or dendrimeric molecule portions. Circular element structures of the ITAG-molecule can be generated for example by reacting two cysteine residues with one another or by reacting one cysteine residue with a thiol ester group within a chain of elements which originally had a linear structure. Branched elements might be prepared for example by synthesizing peptides in which, starting from L-lysine, a new lysine residue is attached to both free amino groups of each of the subsequent lysine residues. It is possible in this way to create a peptide structure with virtually any extent of branching. It is then possible for example for transduction elements and/or allergen elements subsequently to be synthesized or coupled onto the branched peptide basic structure.

Preferred embodiments of the ITAG-molecules according to the present invention are molecules comprising as a transduction element the HIV-tat sequence, preferably the amino acids 46 or 47 to 57 of the HIV-tat sequence. In a more preferred embodiment, the allergen element has a peptide or protein structure containing an allergenic determinant originating from the group comprising insect or plant venom, plant pollen, animal dander, food-allergens, latex, and excrements of arthropods, even more preferred originating from the group of allergens described above in this patent application or most preferred originating from the group of allergens comprising PLA2 (phospholipase A2 allergen from bee venom, also termed Api 1 m), Bet v 1 (birch pollen allergen), Fel d 1 (cat dander allergen), Der p 1 and Der p 2 (house dust, dust mite excrements).

The Fel d 1 allergen consists of two chains, chain 1 and chain 2 forming a heterodimer. For the allergen element of an ITAG-molecule the order of said two chains of Fel d 1 may be chain 1 followed by chain 2 or chain 2 followed by chain 1 when starting from the N-terminus. Of course it is possible that more than one sequence or fragment of a sequence of each chain is present in the allergen element. The chains or fragments are directly covalently linked to one another or are separated by spacer molecules or spacer peptide sequences.

Thus, preferred embodiments of the present invention are ITAG-molecules having a TAT-sequence, preferably the TAT sequence of aa 46 or 47 to 57, or a VP22 sequence or an antennapedia sequence as transudcing element in combination with the Der p 1, Der p 2, PLA2, Bet v 1 or Fel d 1 amino acid sequence. More preferred are ITAG-molecules having the TAT-sequence of aa 46 or 47 to 57 in combination with the Fel d 1 chain 2 and the Fel d 1 chain 1, and, optionally, a His-tag.

Further, the different elements of an ITAG-molecule may be directly linked to one another, e.g. covalently linked, or may be linked via spacer molecules to one another. Preferably short spacer molecules are present between the transduction element and the allergen element. Preferably the spacer elements are short peptides comprising 2 to 10 amino acids, preferably a Glycin-Serine peptide or a Threonin-Serine-Glycine-Serine peptide. If a tag element is present in the ITAG-molecule, said tag element is preferably a HIS-tag.

### Peptides, proteins, amino acids, unusual amino acids, postranslational modifications etc.

The terms peptide and protein are used side by side as equivalent in the present patent application. A peptide or a protein means for the purposes of the invention a covalent connection of at least two amino acids via a peptide linkage. The term "amino acid" and the term "amino acid residue" are used as equivalent in the present application, i.e. the meaning of the two terms is identical. The terms amino acid/amino acid residue and peptide/protein are used in the present application in the form of the widest possible definition.

Amino acids mean for the purposes of the invention besides the 20 amino acids determined by the genetic code also the amino acids which can be encoded by stop codons, such as, for example, seleno-cysteine or pyrro-lysine. Additionally included are all known amino acid and peptide derivatives such as, for example, glycosylated, phosphorylated, sulfated amino acids/peptides, and L-isomeric and D-isomeric amino acids, and amino acid and peptide derivatives which will be known in future. Amino acid and peptide derivatives can arise, or be prepared specifically, by posttranslational modifications, by chemical modifications, by enzymatic modifications or on the basis of other mechanisms. The resulting peptides may comprise modifications which may occur in all regions of the peptide molecule. For example, modifications may occur in the peptide backbone, in the amino acid side chains, at N-terminal ends of the peptide or at C-terminal ends of the peptide. The modifications may be present in single amino acids, in a plurality of amino acids or in all amino acids, and it is possible for no, one or a plurality of types of modifications to be present in any combinations in a peptide. The peptides may be branched, the peptides may be in cyclic form, and any combinations of branched and cyclic peptides are possible. Branched and/or cyclic peptides may arise through natural biological processes or be prepared by synthesis. Examples of unusual amino acids which may be mentioned by way of example are, inter alia: alpha-aminobutyric acid, beta-aminobutyric acid, beta-aminoisobutyric acid, beta-alanine, gamma-aminobutyric acid, alpha-aminoadipic acid, 4-aminobenzoic acid, aminoethylcysteine, alpha-aminopenicillanic acid, allysine, 4-carboxyglutamic acid, cystathionine, carboxyglutamic acid, carboxyamidomethylcysteine, carboxymethylcysteine, cysteic acid, citroline, dehydroalanine, diaminobutyric acid, dehydroamino-2-butyric acid, ethionine, glycine-proline dipeptide, 4-hydroxyproline, hydroxylysine, hydroxyproline, homoserine, homocysteine, histamine, isovaline, lysinoalanine, lanthionine, norvaline, norleucine, ornithine, 2-piperidinecarboxylic acid, pyroglutamic acid, pyrrolysine, proline-hydroxyproline dipeptide, sarcosine, 4-selenocysteine, syndesines, thioproline, etc. All said amino acids can be present in the form of their L isomers or in the form of their D isomers as long as this is permitted by their structure. In general, all currently known amino acids and amino acid derivatives which occur naturally or are formed or can be prepared enzymatic or chemically or in another way, and modifications of amino acids to become known in future, are included in the term "amino acid" and may be constituent of ITAG-molecules for the purposes of the invention.

Examples of postranslational or chemical modifications which may be present in one or more elements of the ITAG-molecule for the purposes of the invention and which are mentioned are, inter alia, modifications of the amino acid sequences by the following structures: binding of free cysteine to a cysteine in the peptide sequence, formation of disulfide linkages between two cysteine residues, methylations, acetylations, acylations, farnesylations, formylations, geranylgeranylations, biotinylations, stearoylations, palmitylations, lipoylations, C-mannosylations, myristoylations, phosphorylations, sulfatylations, N-glycosilations, O-glycosilations, amidations, deamidations, demethylations, cysteinylations, carboxylations, hydroxylations, iodinations, oxidations, pegylations, prenylations, ADP-ribosylations, 5'-adenosylations, 4'-phosphopan-theinations, glutathionylations, covalent bonding: of flavin, of heme groups (or other porphyrins), of nucleic acids or of nucleic acid derivatives, of lipids or of lipid derivatives, of phosphatidylinosistol, of glycosylphosphatidylinositol anchors (GPI anchors), of pyridoxal phosphate, of mannose 6-phosphate, modifications of cysteine to carboxyamidomethylcysteine or carboxymethylcysteine or pyridylethylcysteine, modification of lysine to lipoic acid, modification of glutamine to pyroglutamic acid, addition of amino acids onto peptides by tRNAs, ubiquitin labeling of peptides, branchings of peptides, e.g. in the form of poly-L-lysine, cyclizations of peptides, e.g. by forming disulfide linkages between 2 cystenine residues, etc., possible. Numerous further modifications of proteins which are, inter alia, also archived in databases are described in the literature (Nucleic Acids Res, 2001, 29:199-201). In general, all currently known modifications of peptides which occur naturally or are formed or can be prepared enzymatically or chemically or in another way, and modifications of peptides which will be known in future, are included in the term "peptide" and may be constituent of ITAG-molecules for the purposes of the invention.

### Peptidomimetics

It is additionally possible for the purposes of the invention for one or more amino acids of elements or the complete element or all elements of the ITAG-molecule to be replaced by structures consisting of peptidomimetics. The term peptidomimetic is used in the present application in the form of the widest possible definition. A peptidomimetic is a substance which comprises non-peptide structural elements and is able to imitate or to antagonize the biological effect of the natural parent molecule. Numerous studies dealing in detail with possibilities for using peptidomimetics as replacement for conventional peptide structures are known in the art. It is generally possible for one or more elements of the ITAG-molecule to be composed entirely or partly of peptidomimetics (Curr Opin Chem Biol, 1997, 1:114-9, J Recept Signal Transduct Res, 2001, 21:489-506, J Med Chem, 2001, 44:1938-50). This may have various advantages. Transduction elements may possibly penetrate more efficiently into cells, antigen elements may lead to an enhanced or reduced immune response relative to the immune response to the conventional antigen, or tag elements may have better physicochemical properties, improving their suitability for the isolation and/or detection of the ITAG-molecule, etc. It is additionally possible through the use of peptidomimetics in some circumstances to reduce or increase the in vivo half-life of the ITAG-molecule, to reduce its toxicity, to improve its solubility in hydrophilic or hydrophobic media, and to prolong its in vitro stability and possibly to reduce the costs for synthesizing the peptidomimetic relative to the cost for synthesizing the corresponding conventional ITAG-molecule without peptidomimetic structures incorporated within it. One example of peptidomimetics are Spiegelmers^{®} supplied by NOXXON Pharma AG, Berlin, Germany. This type of peptidomimetics has the advantage for example that they do not elicit an immune response and therefore could be employed in a worthwhile manner for example in transduction elements, tag elements, spacer elements, etc. of ITAG-molecules. Spiegelmers^{®} would, however, not be suitable as antigen element.

### Modification of ITAG-molecules

ITAG-molecules or elements of ITAG-molecules can be modified enzymatically, chemically or by other methods by numerous methods known to the skilled worker. For example, peptides can be provided with phosphorus groups by using kinases, phosphorus groups can be removed using phosphatases, sugar structures can be removed using glycosidases, etc. Appropriate kinases, phosphatases and glycosidases etc., and the appropriate protocols, are obtainable from various manufacturers such as, for example, New England Biolabs, Beverly, MA, USA. Phosphorylation of ITAG-molecules or of elements of ITAG-molecules can additionally be used to label ITAG-molecules or elements of ITAG-molecules with radioactive phosphorus, thus making them easily detectable in vitro and/or in vivo. It is also possible to modify ITAG-molecules or elements of ITAG-molecules by chemical reactions. For example, disulfide bridges can be destroyed by reduction, thioester groups can react covalently with cysteine residues, or two cysteine residues can react to give a disulfide bridge, making it possible to prepare circular or branched ITAG-molecules or elements of ITAG-molecules (e.g. IMPACT™-TWIN Protein Fusion and Purification System, New England Biolabs, Beverly, MA, USA). It is also possible to have a specific influence on modifications of the ITAG-molecule or of elements of ITAG-molecules through the selection of the expression system. For example, no glycosylation takes place in bacteria, and insect cells synthesize only particular types of glycosylations, whereas mammalian cells produce complete glycosylations. It is also possible to use for the expression cell lines which have been modified in such a way, or have been selected in such a way, that they are able specifically to produce, or unable specifically to produce, particular postranslational modifications. These advantageous properties may be inter alia a better solubility in hydrophilic or hydrophobic solvents, a longer stability of the ITAG-molecule, for example, at room temperature, at 37°C, at 4°C, at -20°C, at -70°C or at other temperatures, a longer molecular stability of the ITAG-molecules if they are present alone or mixed with other solid, liquid or gaseous substances, e.g. in the form of preparations as medicament or diagnostic aid, a higher penetrability of the ITAG-molecules for cell membranes, for membranes of organelles, for the blood-brain barrier, for the blood-CSF barrier and for other biological membranes and barriers, etc., a higher or lower in vivo or in vitro half-life, a lower or higher toxicity, a better in vivo or in vitro detectability of the ITAG-molecule etc.

### Peptidomimetics

A further possibility for preparing ITAG-molecules or elements of ITAG-molecules is chemical synthesis of peptides or peptidomimetics (Goodman, M., A. Felix, L. Moroder, and C. Toniolo. 2002. Houben-Weyl: Synthesis of Peptides and Peptidomimetics. Thieme Medical and Scientific Publishers. Vol. 22) or of combinations of peptides and peptidomimetics. The preparation of ITAG-molecules or of elements of ITAG-molecules by chemical synthesis can take place for example by the Merrifield solid-phase synthesis protocol using automatic synthesizers and synthetic chemicals which are obtainable from various manufacturers. An example of a company which supplies syntheses of peptidomimetics is The Peptide Laboratory^{™}, Benicia, CA, USA. Numerous synthons for conventional peptides and for peptidomimetics can be purchased, for example, from Sigma-Aldrich Co, St. Louis, MO, USA.

The peptide portions, amino acid portions, amino acid derivative portions, peptidomimetic portions etc., present in the medicaments and diagnostic aids, of the ITAG-molecules may also be in the form of their salts as long as these salts are pharmacologically acceptable salts. Medicaments or diagnostic aids intended for injection may be for example sterile aqueous or oily solutions which are mixed according to the prior art with suitable excipients such as, for example, dispersants, humectants and agents which stabilize suspensions. The sterile solutions for injection may be produced using pharmacologically acceptable diluents or solvents such as, for example, 1,3-butanediol. Among the acceptable solvents and buffers which can be used are, inter alia, water, Ringer's solution, isotonic sodium chloride solutions etc. In addition, sterile oils, including synthetic mono- or diglycerides, can be used. It is possible in addition to employ fatty acids such as, for example, oleic acid to prepare the solutions for injection. It is furthermore possible to use dimethylacetamide, detergents, including ionic and nonionic detergents, polyethylene glycols, etc. Mixtures of the above mentioned substances are likewise possible. It is additionally possible for medicaments also to be produced in the form of mixtures with biodegradable polymers which release the medicaments continuously. One example of such a system is, for example, the Atrigel system (Atrix Labs, Fort Collins, CO, USA). Liquid preparations intended for injection purposes can be produced from sterile powders or from granules by dissolving in aqueous or nonaqueous solvents. The powders and granules on which these solutions are based may comprise one or more of the substances mentioned for medicaments which can be administered orally. Suitable solvents are, inter alia, water, polyethylene glycol, polypropylene glycol, ethanol, corn oil, cottonseed oil, coconut oil, benzyl alcohol, sodium chloride solutions or various other buffers. Further possible ingredients are colorants, preservatives etc.

The amount of ITAG-molecules and further ingredients of the medicaments depends on the dosage form, dosage frequency, the chosen administration route, the age, sex, weight and the state of health of the patient, etc. An additional factor to be taken into account is whether the treatment is carried out for diagnosis, therapy or for prophylaxis. Numerous works on the formulation and dosage of medicaments are known to the skilled worker (Hover, J.E. 1975. Remington's Pharmaceutical Sciences. Mack Publishing Co., Easton, PA, USA, Libermann, H.A., and L. Lachman. 1980. Pharmaceutical Dosage Forms. Marcel Decker Inc., New York, NY, USA).

A preferred embodiment of the invention is the intranodal injection of ITAG-molecules. Intranodal injections are injections directly into lymph nodes, which bring the allergen element directly to a region within the body, which region is responsible to a large extend for antigen processing and presentation, thereby further improving the effectiveness of ITAG-molecules (WO 02/28429). Preferred lymph nodes for intranodal injections of ITAG-molecules are the major lymph nodes located in the regions of the groin, the underarm and the neck, preferably the lymph nodes located in the region of the groin, most preferably the axillary or inguinal lymph nodes.

In addition, the injections may injections being adjacent ot the lymphatic tissue, in particular, adjacent to the lymph node.

### Possibilities for examining the efficacy of ITAG-molecules

Various in vitro and in vivo experiments can be carried out to examine the efficacy of ITAG-molecules in relation to desensitisation of an individual suffering from allergy. Suitable as in vitro model are, for example, peripheral blood mononuclear cells (PBMCs), e.g. obtained from the blood of patients suffering from allergic disorders. The advantage of such cells is that the exact antigen against which the particular patient has an allergic response is frequently known. This knowledge makes it possible for example to simulate a desensitisation of the patient in vitro, before clinical studies are carried out on the patient or in experimental animals. For this purpose, for example, the PBMCs from allergic individuals, preferably from allergic humans can be incubated with the particular antigen against which the allergic individual reacts, or with a ITAG-molecule which comprises at least one peptide or protein structure comprising the allergenic determinant of the allergen towards the individual mounts an allergic reaction. Thus, the immunological reaction of the individuals cells to various dosage forms (complete ITAG-molecule, molecules with/without transduction element, molecules with/without targeting element etc.) of the allergenic determinant can be investigated. Suitable measurement parameters are inter alia cytokine determinations in the cell culture supernatant. Various types of T cells are involved in the immune response to an antigen, such as, for example, T-helper cells of type 1 (Th1 cells) or of type 2 (Th2 cells) or of type 0 (Th0 cells). The type of T cells involved in each case has a great effect on whether the immune response induced against the antigen primarily consists of immunoglobulins of class E (IgE) or of immunoglobulins of class G (IgG). It is known from the literature that, in particular, IgE immune responses are responsible for allergic reactions and asthma, whereas IgG immune responses are usually associated with a tolerance to the antigen. The particular T cell type activated by the treatment of the PBMCs with an antigen can be determined for example also by determining the expression of surface antigens on the cell surface or by determining messengers such as, for example, cytokines which are released by the PBMCs. Markers of a Th1 immune response which can be determined are, for example, interferon gamma (INF-g) or interleukin-1 (IL-1) in the cell culture medium, whereas IL-4, IL-5, IL-6 and IL-13 indicate a Th2 immune response. These cytokines can be detected by standard methods known to the skilled worker, such as, for example, ELISA determinations from, for example, the cell culture supernatants or FACS analyses of the messengers present on the surface or inside the cells of the PBMCs, or by Western blots of cell culture supernatants or cell lysates etc. Numerous other methods suitable for detecting these or other messengers are disclosed in the literature. Besides the messengers released by the PBMCs, it is also possible to use intracellular or membrane-associated messengers or further proteins for immunological characterization of the T cells in PBMCs. Numerous antibodies suitable for such investigations are supplied inter alia by Pharmingen, San Diego, CA, USA, Beckmann Coulter Inc., Fullerton, CA, USA, Santa Cruz Biotechnology Inc., Santa Cruz, CA, USA, etc. Corresponding investigations can, however, be carried out not only with patients' primary cells but also with cells obtained from appropriately treated experimental animals, e.g. mice, rats, guinea pigs, etc. Experiments with experimental animals have the advantage that not only can the cells of these animals be studied in vitro, but that the immune system can be investigated in vivo in the context of an intact organism. It is thus also possible to investigate the effect of the dosage, composition and administration form of the ITAG-molecules and of corresponding controls such as, for example, molecules which consist only of an antigen or which consist only of a transduction element. It is possible to investigate inter alia whether there are differences in the nature of the immune response if ITAG-molecules or corresponding controls are injected subcutaneously in a conventional way or if the injection takes place for example directly into lymph nodes. A further investigation which can be carried out in experimental animals is administration of ITAG-molecules or corresponding controls with and without simultaneous administration of adjuvants such as, for example, mineral oil, mycobacterial extracts or Freund's adjuvant. It is also possible to ascertain the most effective or best-tolerated time schedule for the allergy shots to be carried out, and the dose and number of allergy shots. Different antigen elements can moreover be tested for their efficacy. It is possible in this way to ascertain the best desensitisation strategy for later studies on human patients.

Besides messengers, intracellular proteins and surface proteins, also suitable in particular for characterizing the immune reaction of cells cultivated in vitro or for characterizing the immune reaction of experimental animals or of human patients in clinical studies are immunoglobulins. It is possible for example by using ELISAs to investigate and quantify whether the antibodies released owing to the administration of the ITAG-molecule are of the IgE type or of the IgG type. This information would indicate the type of immune reaction involved.

Since the messengers released by the T cells also inter alia have an effect on the proliferation of cells of the immune system which are present inter alia in PBMC preparations, it is also possible to characterize the immune response by determining the proliferation of cells such as, for example, PBMCs. It is possible for this purpose to carry out for example in vitro investigations such as, for example, DNA incorporation studies with tritium-labeled thymidine to detect cell growth. Numerous other methods known to the skilled worker for determining cell proliferation can likewise be used in these investigations. Proliferation of certain cells of the immune system can also be determined in vivo by for example carrying out FACS investigations with blood samples from experimental animals or human individuals taking part in studies. It is possible by selecting suitable antibodies to quantify for example different subpopulations of cell types present in the blood. The effect of a treatment with an ITAG-molecule or corresponding controls can be investigated in this way.

For evaluation of ITAG-molecules in clinical studies exposure test to determine the patient's response to allergens can be used. Examples for such exposure tests known in the art are skin prick tests, conjunctival provocation tests, rhinomanometry, antigen capture tests, pulmonary function test or metacholin tests. These exposure tests can be done in comparison between patients treated with conventional commercial allergen preparations such as cat dander extracts, patients treated with recombinant or native proteins such as Fel d 1 and patients treated with recombinant ITAG-molecules such as for example ITAG-molecules with the complete Fel d 1, or ITAG-molecules with fragments of Fel d 1. Similar comparisons can be done with other antigens such as Bet v 1, Der p 1, Der p 2 or PLA2. The patient groups tested for example can be patients with a single allergy to the specific allergen tested, control patients with a single allergy to another allergen different to the tested allergen and control patients without any allergy. It is also possible to include groups of patients having allergies towards multiple allergens of which one allergen is the allergen to be tested, or of which no allergen is the allergen to be tested (=control group). It is also possible to include groups of patients, which patients were treated with conventional anti-allergy therapies in the past without success towards the allergen to be tested in the clinical study. The clinical study can be done in different groups of the population regarding age (adolescent versus adult, individuals 5 years or younger and individual older than 5 years), sex (male versus female), ethnic groups (African Americans, Asian Americans, Caucasian Americans, etc.), etc.. The studies are preferably done as double-blind placebo-controlled, prospective single or multiple center studies. To ensure the "double-blinded" status of the study the placebo group can be treated with substances mimicking the effects of an immunization by provoking local reactions by injection of histamine dihydrochloride as placebo. This ensures that the medical doctors conducting the blinded study can not distinguish the placebo- from the test-group. The success of the treatment can be determined by various methods known in the art, such as "quality of life" questionnaires, reduction of medication needed to treat allergy symptoms (e.g. reduction of use of anti-histamine or cortisone), reduction in drop out rate in the course of the study (the more successful the treatment, the more likely it is, that the patients freely stay in the study), etc. In addition the success of the study can be determined by measuring laboratory parameters such as Interferon gamma (IFN-g), Interleukin (IL)-4, IL-5, IL-2, IL-10, etc. measuring allergen-specific antibodies such as IgG1 or IgG4 titers, or measuring allergen-mediated leukotriene-secretion of Leukocytes of allergic patients using the CAST® test.

The method according to the present invention comprising the step of administering ITAG-molecules to an individual for desensitisation is particularly useful for desensitizing individuals against an allergen where former parenteral, in particular, subcutaneous desensitisation against said allergen was not effective.

Further, the method according to the present invention may be conducted for desensitizing individuals which display adverse events to solvents or adjuvants used in medicaments for desensitisation via the parenteral, in particular via the subcutaneous or sublingual route.

### Exemplary embodiments

The following exemplary embodiments are intended to illustrate the invention by way of example but are by no means intended to restrict the range of protection of the invention.

### Example 1: Expression and purification of ITAG-molecules

The expression and isolation of ITAG-molecules and of the corresponding recombinant allergen proteins or peptides without fusion to a transduction element (e.g. HIV-tat sequence) was carried according to standard procedures known in the art. A bacterial vector was prepared containing a recombinant construct of either an allergen element, e.g. Fel d 1, PLA2, etc. and as a transduction element the HIV-tat sequence representing the amino acid sequence fragment GYGRKKRRQRRRG of HIV-tat N-terminally fused to the allergen element. All constructs were inserted into the pQE-30 expression vector (Qiagen, Hilden, Germany) and contained an N-terminal His-tag, which subsequently was used for purification of the proteins. The expression and isolation of ITAG-molecules and of the corresponding recombinant allergens, e.g. rFel d 1, rPLA2, etc. was carried out in accordance with the manufacturer's instructions for the pQE-30 expression vector and the Ni-NTA Superflow matrix (both from Qiagen, Hilden, Germany). In detail, a preculture of E. coli M15 bacteria transfected with the particular expression vector (pQE-30-ITAG molecule, pQE-30-allergen) were set up in 20 ml of medium (2x YT medium, 100 µg/ml ampicillin, 25 µg/ml kanamycin) in a bacterial shaker at 37°C overnight. The precultures were then cultivated in 2000 ml of medium (2x YT medium, 100 µg/ml ampicillin, 25 µg/ml kanamycin) at 37°C in a bacterial shaker until they reached an optical density of 0.6 at a wavelength of 600 nm. After addition of IPTG at a final concentration of 1 mM and a further growth phase of from 4 to 15 h, the bacteria were separated from the culture medium by centrifugation at 2000 g for 20 minutes. The bacterial pellets were stored at -20°C. Cell lysates were produced by thawing the bacterial pellet, resuspending in 8 M urea solution (5 ml of urea solution per gram wet weight of the bacterial pellets), cautiously stirring for 1 to 2 h and subsequently centrifuging at 48000 g for 30 min. The clear supernatant was used for preparative nickel chelate chromatography of the recombinant. The recombinant molecules were isolated using 49 or 18 ml columns (Bio-Rad Laboratories Inc., Hercules, CA, USA), packed with 5 to 10 ml of Ni-NTA Superflow matrix (Qiagen) and a chromatography system from Bio-Rad (Econo pump and UV monitor). The chromatography column was initially washed with 5 column volumes of buffer A (100 mM NaH2PO4, 10 mM Tris/HCl, 6 M guanidine HCl, pH adjusted to 8.0 with HCl) and then the bacterial lysate was loaded onto the column at a flow rate of 1.4 ml/min. Then 5 to 10 column volumes each of buffer A and buffer B (buffer B: 100 mM NaH2PO4, 10 mM Tris/HCl, 8 M urea, pH 8.0) are pumped onto the column at a flow rate of 1.4 ml/min, and the absorption of the flow-through at a wavelength of 280 nm (A280) was observed. As soon as the flow-through reached a stable A280 of less than about 0.01, the column was washed with 5 to 10 column volumes of buffer C (100 mM NaH2PO4, 10 mM Tris/HCl, 8 M urea, pH adjusted to 6.3 with HCl) until finally a stable A280 of less than about 0.01 was reached. The recombinant molecules were then eluted with buffer E (100 mM NaH2P04, 10 mM Tris/HCl, 8 M urea, pH adjusted to 4.5 with HCl). Quantification of the recombinant molecules was done by conventional Bradford protein assays as known in the art. Purity of the recombinant molecules was determined by SDS- polyacrylamide gel electrophoresis as known in the art.

### Example 2: Allergen-induced stimulation of PBMCs of allergic patients

The objective of this evaluation is to investigate if an allergen construct is able to stimulate in vitro the proliferation of peripheral blood mononuclear cells (PBMC) prepared from an individual sensitised against (allergic to) the allergen contained in the construct tested. All study protocols followed the obligations of the local ethics committee. Freshly drawn, heparinized blood is mixed 1:1 with room temperature PBS. For every 10 ml blood/PBS mixture 3 ml of Ficoll solution is added to a 50 ml conical Tube and carefully overlayed with the blood/PBS mixture, centrifuged in a Beckman GPR centrifuge using a GH 3.7 horizontal rotor 30 minutes at 2000 rpm (900x g) at room temperature without using a brake. Subsequently the top layer of plasma/PBS is aspirated and the intermediate layer containing the PBMCs is carefully transferred into a fresh 50 ml tube. The tube is filled with PBS and centrifuged for 10 minutes with 1300 rpm (400x g) at room temperature using a brake. This step is repeated once. The resulting cell pellet is resuspended in a small volume of complete RPMI and stored on ice until further used. The cytospin 2 funnel is assembled according to the manufacturer's instructions and 100 µl of cell suspension in RPMI medium, containing about 100000 cells are put into the funnel. After centrifugation at speed 25 for time 2 (cytospin 2 specifications) the funnel is dissembled and the slide with the cells is air dried shortly. The cells are fixed for 5 sec. using fixing solution, stained for 15 sec. in eosin solution, stained 30 sec. using haematoxylin solution and finally washed with tap water and air dried. The cells are inspected for quality and purity using a light microscope. All solutions are according to the cytospin manufacturer's specifications.

### Antigen-stimulation of PBMCs:

After cell counting, PBMCs were adjusted to 1 000 000 cells/ml with complete RPMI and 100 µl/well of cell suspension added into 96-well microtiter tissue culture plates. Subsequently antigen or control stock-solutions were transferred into the wells resulting in 0.01, 0.1, 1, 10, 100 and 1000 nM final concentration of the antigen or controls. The antigen was either a construct with the following structure: N-terminus, His-tag, HIV-Tat, antigen, C-terminus. The test substances contained as an antigen either a Fel d 1 fusion protein (with the structure: N-terminus, chain 2 of Fel d 1, chain1 of Fel d 1, C-terminus) or PLA or Bet v1 or Der p1 instead of Fel d 1. The controls contained either only buffer or the antigen without any additional peptides fused to it. i.e. without the HIV-TAT. All stimulations were done in triplicates (3 wells of a 96-well plate). The tissue culture plates were incubated for 6 days at 37°C and 5% CO2 in a humidified cell culture incubator.

### Example 3: Proliferation assay of antigen-stimulated PBMCs of allergic patients

On the day of the 3H-Thymidin-incorportion assay 20 µl/well of a 50 µCi/ml 3h-Thymidine solution in complete RPMI medium is added to the cells resulting in a final dose of 1 µCi/well. 6 or 18 h later cells are harvested employing a multi-well harvester. Each well of the 96-well tissue culture plate is filled, aspirated and transferred to a paper filter ten times. This ensures complete transfer of the cells for subsequent counting. Finally the filter papers are washed with 100 % ethanol to remove any non-incorporated radioactivity, transferred to scintillation vials, scintillation fluid is added and the vials are measured.

### Analysis

All measurements were done with triplicates of samples for each concentration and each kind of antigen. Mean values +/- standard deviation of scintillation counts of the three samples were calculated and graphically displayed using standard Microsoft Excel spreadsheet software.

### Example 4: Cytokine-release of allergen-stimulated PBMCs of allergic patients

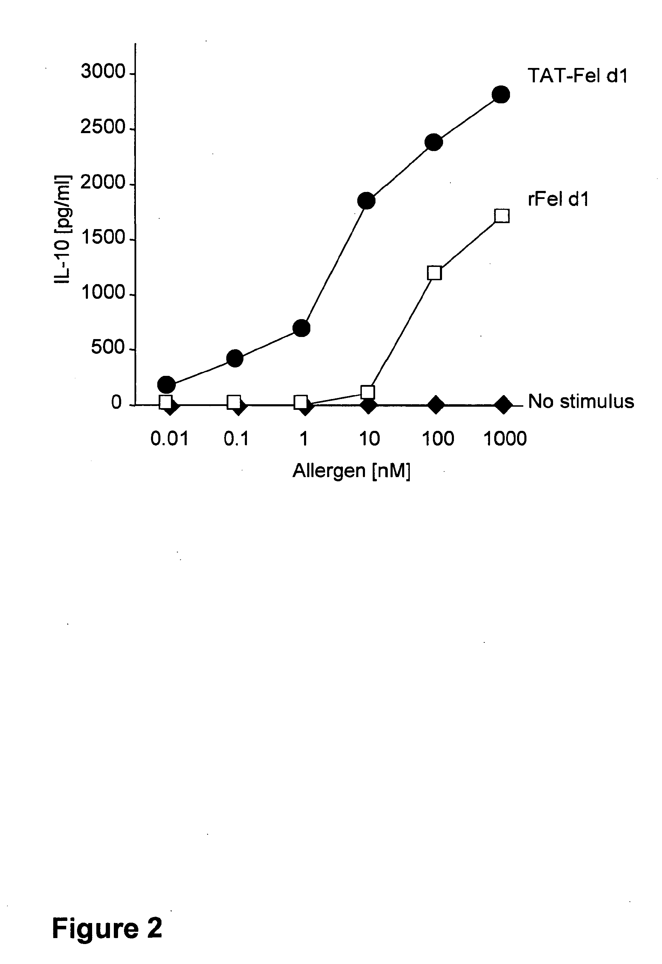

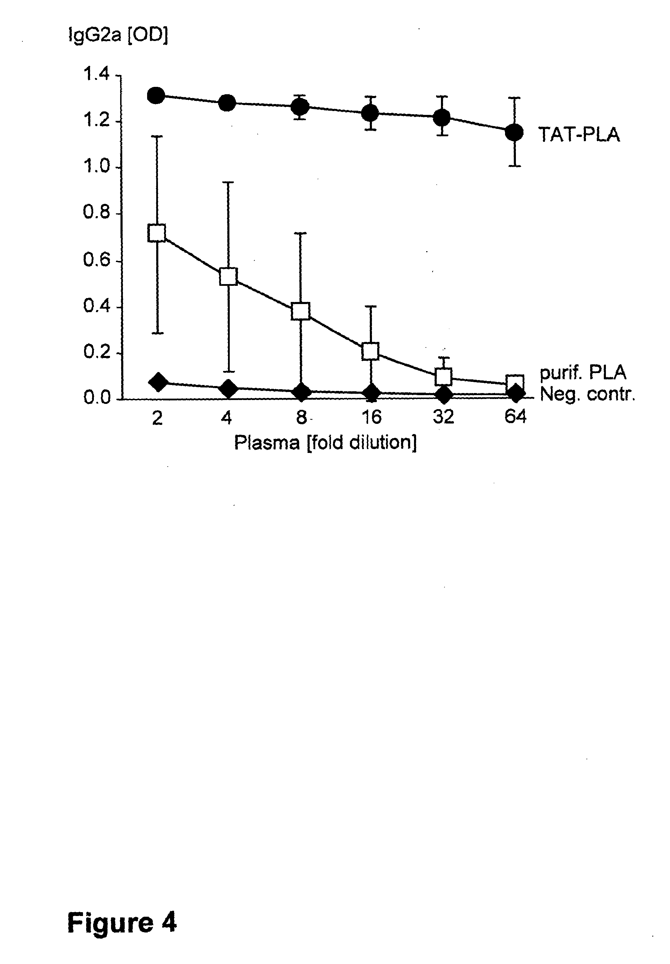

ITAG-molecule-stimulated cytokine release
PBMCs were isolated from the blood of allergic patient as described in example 2 and diluted 10Exp6/ml of medium. 0.1 mL portions of this cell suspension were seeded in 96-well plates and treated with from 0.01 to 1000 nM of the isolated antigens for 5 days. The cell culture medium was not replaced in this time. After centrifugation of the 96-well plates, the supernatants were removed and stored at -20°C until the cytokines were analyzed. The PBMCs were treated with the Fel d 1 antigen against which the patient from whom the PBMCs were isolated shows an allergic reaction. The INFg and IL-10 immunoassays (ELISAs = enzyme-linked immunosorbent assays) were carried out by methods known to the skilled worker using DuoSet® ELISA Development Systems from R & D Systems Inc., Minneapolis, USA according to the manufacturers instructions. Figure 1 and 2 show the results obtained. Using TAT-Fel d 1 about 10-fold less allergen is necessary to induce the same INFg secretion as compared to rFel d 1 (recombinant Fel d 1 without a transduction element, in this case HIV-tat) (Figure 1). INFg is the predominant cytokine secreted by Th1 cells and induces the production of allergy-protective IgG antibodies. This increase IgG antibody response has been confirm in in-vivo experiments (see Fig.4). Therefore TAT-Fel d 1 induces a more allergy-protective immune response as compared to rFel d 1. Figure 2 shows, that TAT-Fel d 1 stimulation also induces a stronger IL-10 response, as compared to rFel d 1 stimulation. According to the present knowledge, production of IL-10 is required for a successful allergen-specific immunotherapy, since induction of allergen-specific tolerance in peripheral T Cells is induced by IL-10. Consequently the increased IL-10 secretion obtained by TAT-Fel d 1 stimulation, as compared to rFel d 1 also indicate, that TAT-Fel d 1 is superior for desensitizing allergic patients.

### Example 5: Allergen-mediated leukotriene-secretion of Leukocytes of allergic patients measured by ELISA

This assay was done according to the manufacturers instructions of the Cellular Antigen Stimulation Test, CAST®, (Bühlmann Laboratories AG, Allschwil, Switzerland). In the CAST® , sedimented leukocytes from allergic patient blood are simultaneously primed with the cytokine IL-3 and stimulated with allergens. Basophilic cells among others generate the allergic mediator, sulfidoleukotriene LTC4, and its metabolites LTD4 and LTE4. These freshly synthesized sulfidoleukotrienes (sLT) are subsequently measured in an ELISA test (Enzyme Linked ImmunoSorbent Assay).

Isolation of Leukocytes: Dextran is added to freshly drawn blood of a patient allergic to cat dander in the presence of an anticoagulant in order to increase the blood viscosity. After 90 minutes at room temperature, the erythrocytes are sedimented, whereas leukocytes and thrombocytes stay in the plasma fraction. Subsequently, the blood supernatant was transferred into separate tubes and the leukocytes were sedimented in a brief centrifugation step. The plasma supernatant containing >90% of the thrombocytes was discarded and the leukocyte pellet resuspended in the stimulation buffer provides with the CAST® kit, which stimulation buffer contained IL-3.

Cell Stimulation: The purified leukocytes of allergic patients were tested for the basal level release of leukotriene (= Neg. contr. = negative control) and for the release after stimulation with an anti-IgE receptor antibody (= Pos. contr. = positive control). The cells were stimulated at 37°C using different Fel d 1-stimuli at concentrations of 20, 2, 0.2 and 0.02 ng/ml final Fel d 1. The stimuli tested were a standarsized cat dander extract (Stallergenes, Kamp-Lintfort, Germany), recombinant Fel d 1 (rFel d 1; Fel d 1 is the major allergen present in cat dander) and an ITAG-molecule comprising HIV-tat as transduction element and Fel d 1 as allergen element (TAT-Fel d 1). The standarsized cat dander extract was used in the samples in an amount resulting in the same final Fel d 1 concentration in the cat dander extract stimulations as compared to the rFel d 1 and TAT-Fel d 1 stimulations. After 40 minutes the cells were centrifuged and the cell supernatant was either frozen for subsequent analysis or immediately tested for Leukotriene using the ELISA provided with the CAST® kit (Bühlmann Laboratories AG, Basel, Switzerland).

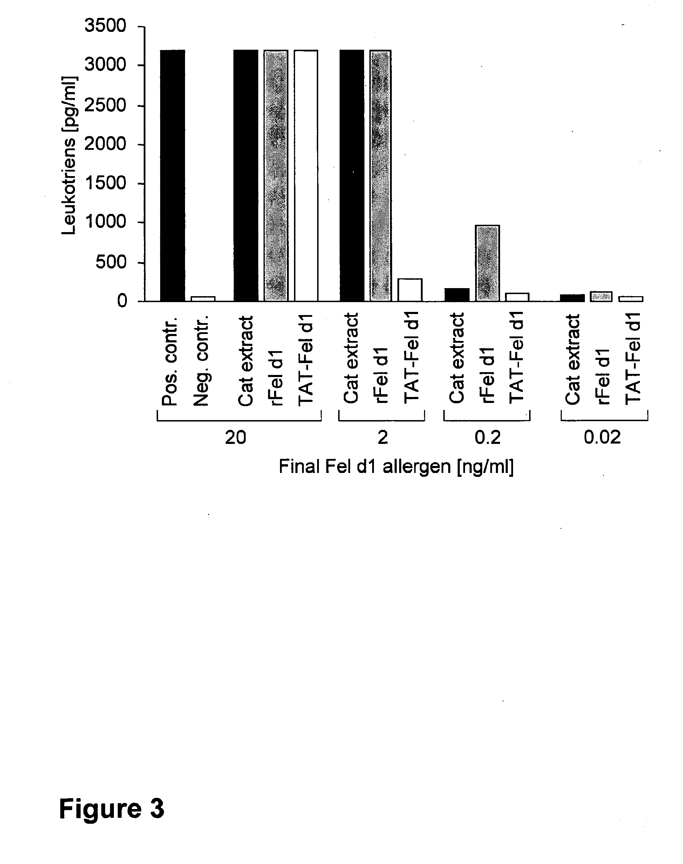

Leukotriene Determination: the ELISA was performed according to the manufacturers instructions. 0.05 mL enzyme label and 0.05 mL antibody solution provided with the CAST® kit were are added to 0.1 mL cell-stimulation supernatants as well as to the leukotrien standards and the controls and incubated for 2 hours at 18-28°C in precoated microtiter plates. After three washing steps, 0.2 mL of the substrate solution (pNPP, para-nitrophenylphosphate) provided by the manufacturer of the CAST® is added to each well and incubated for another 30 minutes at 18-28°C on a rocking platform. Finally 0.05 mL of the stop solution provided in the kit is added to each well and the absorbance is measured at 405 nm in a microtiter plate reader. The standard values are used to calculate the leukotriene concentrations of the controls and samples. The results of the experiment are shown in figure 3. A final Fel d 1 concentration of 2 ng/mL results in a leukotriene signal above the detection limit of the ELISA for the positive control and for the stimulation supernatant obtained with rFel d 1, whereas TAT-Fel d 1 results in leukotrien release only slightly above the background of the negative control and with 0.2 ng/mL final Fel d 1 stimulus the rFel d 1-stimulated cells release about 10-time more leukotrienes, as compared to TAT-Fel d 1-stimulated cells of cat dander allergic patients. Consequently the TAT-Fel d 1 ITAG-molecule results in clearly less release of leukotrienes, which represent an important factor of adverse side effects observed during desensitisation of allergic patients.

### Example 6: In vivo IgG2a level in intranodal immunised mice

### Immunization of mice

In order to test the efficacy of ITAG-molecules, CBA/2 mice were immunized 3 times at an interval of 2 weeks each time with recombinant ITAG-molecules together with aluminum hydroxide as adjuvant in a manner known to the skilled worker. The recombinant ITAG-molecules were produced as described in example 1. For the intranodal injection, the tissue of the inguinal lymph node was exposed surgically so that direct injection was possible. The ITAG-molecule used was a protein consisting of the HIV Tat sequence as transduction element and the PLA2 (phospholipase A2 from bee venom, also termed Api 1 m) peptide as allergen element (designation: TAT-PLA2). PLA2 purified from bee venom purchased from Sigma-Aldrich, Taufkirchen, Germany (designation: purify. PLA2) was used as control, and 0.9% strength saline solution (designation: Neg. control) was used as negative control. The intranodal immunization took place with 0.1 µg of ITAG-molecule or corresponding amounts of control protein or control buffer together with aluminium hydroxide as adjuvant. Animals were immunized in each experimental group. Blood was taken from the tail vein of each experimental animal before the first immunization and subsequently after 2, 4 and 6 weeks. The blood was allowed to coagulate at room temperature, and the serum was obtained after centrifugation and stored at -20°C until analyzed. The sera produced in this way were used to determine the PLA2-specific IgG2a titers using an enzyme-linked immunosorbent assay (ELISA).

### Determination of the PLA2-specific IgG2a titers in mouse sera

For determination of the PLA2-specific IgG2a titer, microtiter plates (96 cavities) were coated with 100 µl/cavity of a solution of 5 µg/ml PLA2 (Sigma-Aldrich, Buchs DG, Switzerland) in carbonate buffer at 4°C overnight. After washing 2x with phosphate-buffered sodium chloride solution (PBS), 0.05% Tween, free protein-binding sites were blocked by incubation with blocking buffer (PBS, 2.5% skim milk powder), 200 µl/cavity, at room temperature for 1 to 2 h. Washing 2× was repeated, and then serial 1:2 dilution series in blocking buffer (50 µl/cavity) of the serum samples to be tested (1:2 to 1:64 dilutions) were incubated at room temperature for 3 h or at 4°C overnight. Incubations without serum or with serum from untreated animals were carried out as negative controls. This was followed by washing 5× and incubating with a 1:500 dilution in blocking buffer of a biotin-labelled anti-mouse IgG2a (PharMingen GmbH, Hamburg, Germany) at room temperature (100 µl/cavity) for 2 h, and again washing 5x. Finally, 100 µl/cavity of a horseradish peroxidase diluted 1:1000 in blocking buffer were incubated at room temperature for 1 h and then washed 6×. The colour reaction was carried out with 100 µl/cavity of a solution of ABTS (2,2'-azinodi-(3-ethylbenzothiazolinesulfonic acid) in ABTS buffer in accordance with the manufacturer's information with 0.1% (v/v) of a 30% strength hydrogen peroxide solution. After about 30 minutes, the absorption at a wavelength of 405 nm (reference filter: 595 nm) was measured. The results of these tests are depicted in figure 4.

## Claims

1. Use of an ITAG-molecule, comprising at least one protein or peptide structure containing an allergenic determinant, and at least one transduction element able to translocate the molecule across membranes, in particular cell membranes from the extracellular space into the interior of a cell, in the manufacture of a medicament adapted for intralymphatic application for desensitizing an individual to an allergen containing said allergenic determinant, against which said individual mounts an allergic response.

2. The use according to claim 1, wherein said at least one transduction element comprises at least one of the following elements or at least one functional fragment thereof selected from the group consisting of HIV-tat, antennapedia, VP22, or inverted sequences thereof, linear, or branched polyarginine, polylysine, or mixed polyargenine/lysine sequences containing at least 4 to 20 arginine and/or lysine amino acids residues.

3. The use according to claim 2, wherein the transduction element is HIV-tat or a functional fragment thereof.

4. The use according to any one of the preceding claims, wherein said protein or peptide structure containing said allergenic determinant is derived from a composition containing allergens selected from the group consisting of insect or plant venom, plant pollen, animal dander, food-allergen, excrements of arthropods, and latex.

5. The use according to any one of the preceding claims wherein said protein or peptide structure containing an allergenic determinant is derived from at least one amino acid sequence selected from Seq.-ID 1 to 8 (Fel d 1, Bet v 1, Der p 1, Der p 2, PLA2) or a fragment thereof.

6. The use according to claim 5 wherein said peptide or protein structure containing an allergenic determinant is selected from Seq.-ID 7 (Fel d 1, chain 2), Seq.-ID 6 (Fel d 1, chain 1), Seq._ID 8 (Seq.-ID 7 N-terminally fused to Seq.-ID 6), Seq.-ID 1 (Seq.-ID 6 N-terminally fused to Seq.-ID 7), a fragment of Seq.-ID 1 or 6 to 8, or of a fusion of a fragment of Seq.-ID 7 fused to a fragment of Seq.-ID 6.

7. The use according to any one of the preceding claims wherein said at least one transduction element and said at least one peptide or protein structure containing an allergenic determinant are interconnected via a linker.

8. The use according to any one of the preceding claims wherein said medicament further comprises a pharmaceutically acceptable carrier and/or a pharmaceutically acceptable adjuvant.

9. The use according to any one of the preceding claims wherein said medicament is adapted for direct injection into lymph nodes having a size/diameter of at least 2 mm.

10. The use according to any one of the preceding claims wherein said medicament comprises a dose of from 0.0001 mg to 0.05 mg of said ITAG-molecule.

11. The use according to any one of the preceding claims for desensitising an individual against an allergen, which individual has not been previously subjected to any desensitisation treatment.

12. The use according to any one of claims 1 to 10 for desensitising an individual against an allergen, which individual has previously been subjected to a desensitisation treatment.

13. The use according to any one of the preceding claims whereby said medicament is administered in between 1 to 10 times to said individual to achieve desensitisation against said allergen.

14. The use according to claim 13 whereby said medicament is administered in between 2 to 5 times to said individual to achieve desensitisation against said allergen.

15. The use according to any one of the preceding claims, whereby said medicament is administered in intervals of several months or years to maintain the desensitisation achieved by previous administrations.

16. The use according to claim 15, whereby said administration to maintain the desensitisation is performed by the subcutaneous route, the sublingual route or any other route different to an intralymphatic route.

17. The use of a molecule as described in any one of the preceding claims in the manufacture of a medicament for decreasing or suppressing the IgE response and/or for increasing the IgG response specific for the allergenic determinant present in said molecule and/or for decreasing the allergen-mediated histamine, leukotriene, tosyl-L-arginine methyl esterase (TAME), prostaglandine G2, and/or pro-inflammatory cytokine release in a human individual.

18. A kit for desensitisation of an individual comprising:
(i) instructions for injection of a medicament comprising an ITAG-molecule into lymphatic tissue of an individual, and/or
(ii) a single or dual chambered syringe, and
(iii) a medicament as defined in any one of claims 1 to 10.

19. A method for desensitizing an individual to an allergen against which said individual mounts an allergic response, using an ITAG-molecule, comprising at least a protein or peptide structure containing an allergenic determinant, and at least one transduction element able to translocate the molecule across membranes, in particular cell membranes from the extracellular space into the interior of a cell, comprising the step of delivering said ITAG-molecule directly into lymphatic tissue of said individual.

20. The method according to claim 19 wherein the ITAG-molecule is a molecules as defined in any one of claims 1 to 10

21. The method according to any one of claim 19 or 20, wherein the step of delivering said ITAG-molecule is carried out at least twice, preferably 2 to 5 times.

22. The method according to any one of claims 19 to 21 wherein said individual has not been previously subjected to any desensitisation treatment.

23. The method according to any one of claims 19 to 21 wherein said individual has been previously subjected to a desensitisation treatment.

24. The method according to any one of claims 19 to 23 whereby said medicament is administered in intervals of several months or years to maintain the desensitisation achieved by previous desensitisation.

25. The method according to claim 24 whereby the administration to maintain the desensitisation is performed by the subcutaneous route, the sublingual route or any other route different to the intra lymphatic route.

26. The method according to any one of claims 19 to 25, wherein said individual is a mammal, preferably a human individual.

27. The method according to any one of claims 19 to 26, wherein said ITAG-molecule is delivered into lymphatic tissue with a single or dual chambered syringe.


SEQUENCE LISTING

<110> Imvision GmbH

<120> Modulation of the immune response by administration of
Intralymphatic Transduction AllerGen (ITAG)-molecules

<130> 3140-19

<160> 13

<170> PatentIn version 3.3

<210> 1
<211> 162
<212> PRT
<213> felis domesticus

<220>
<221> MISC_FEATURE
<223> Fel D 1, chain 2+1

<400> 1

Val Lys Met Ala Glu Thr Cys Pro Ile Phe Tyr Asp Val Phe Phe Ala
1               5                   10                  15

Val Ala Asn Gly Asn Glu Leu Leu Leu Asp Leu Ser Leu Thr Lys Val
            20                  25                  30

Asn Ala Thr Glu Pro Glu Arg Thr Ala Met Lys Lys Ile Gln Asp Cys
        35                  40                  45

Tyr Val Glu Asn Gly Leu Ile Ser Arg Val Leu Asp Gly Leu Val Met
    50                  55                  60

Thr Thr Ile Ser Ser Ser Lys Asp Cys Met Gly Glu Ala Val Gln Asn
65                  70                  75                  80

Thr Val Glu Asp Leu Lys Leu Asn Thr Leu Gly Arg Glu Ile Cys Pro
                85                  90                  95

Ala Val Lys Arg Asp Val Asp Leu Phe Leu Thr Gly Thr Pro Asp Glu
            100                 105                 110

Tyr Val Glu Gln Val Ala Gln Tyr Lys Ala Leu Pro Val Val Leu Glu
        115                 120                 125

Asn Ala Arg Ile Leu Lys Asn Cys Val Asp Ala Lys Met Thr Glu Glu
    130                 135                 140

Asp Lys Glu Asn Ala Leu Ser Leu Leu Asp Lys Ile Tyr Thr Ser Pro
145                 150                 155                 160

Leu Cys

<210>  2
<211>  160
<212>  PRT
<213>  betula verrucosa

<220>
<221>  MISC_FEATURE
<223>  Bet v 1

<400>  2

Met Gly Val Phe Asn Tyr Glu Thr Glu Thr Thr Ser Val Ile Pro Ala
1               5                   10                  15

Ala Arg Leu Phe Lys Ala Phe Ile Leu Asp Gly Asp Asn Leu Phe Pro
            20                  25                  30

Lys Val Ala Pro Gln Ala Ile Ser Ser Val Glu Asn Ile Glu Gly Asn
        35                  40                  45

Gly Gly Pro Gly Thr Ile Lys Lys Ile Ser Phe Pro Glu Gly Phe Pro
    50                  55                  60

Phe Lys Tyr Val Lys Asp Arg Val Asp Glu Val Asp His Thr Asn Phe
65                  70                  75                  80

Lys Tyr Asn Tyr Ser Val Ile Glu Gly Gly Pro Ile Gly Asp Thr Leu
                85                  90                  95

Glu Lys Ile Ser Asn Glu Ile Lys Ile Val Ala Thr Pro Asp Gly Gly
            100                 105                 110

Ser Ile Leu Lys Ile Ser Asn Lys Tyr His Thr Lys Gly Asp His Glu
        115                 120                 125

Val Lys Ala Glu Gln Val Lys Ala Ser Lys Glu Met Gly Glu Thr Leu
    130                 135                 140

Leu Arg Ala Val Glu Ser Tyr Leu Leu Ala His Ser Asp Ala Tyr Asn
145                 150                 155                 160

<210>  3
<211>  222
<212>  PRT
<213>  Dermatophagoides pteronyssinus

<220>
<221>  MISC_FEATURE
<223>  Der p 1

<400>  3

Thr Asn Ala Cys Ser Ile Asn Gly Asn Ala Pro Ala Glu Ile Asp Leu
1               5                   10                  15

Arg Gln Met Arg Thr Val Thr Pro Ile Arg Met Gln Gly Gly Cys Gly
            20                  25                  30

Ser Cys Trp Ala Phe Ser Gly Gly Ala Ala Thr Glu Ser Ala Tyr Leu
        35                  40                  45

Ala His Arg Asn Gln Ser Leu Asp Leu Ala Glu Gln Glu Leu Val Asp
    50                  55                  60

Cys Ala Ser Gln His Gly Cys His Gly Asp Thr Ile Pro Arg Gly Ile
65                  70                  75                  80

Glu Tyr Ile Gln His Asn Gly Val Val Gln Glu Ser Tyr Tyr Arg Tyr
                85                  90                  95

Val Ala Arg Glu Gln Ser Cys Arg Arg Pro Asn Ala Gln Arg Phe Gly
            100                 105                 110

Ile Ser Asn Tyr Cys Gln Ile Tyr Pro Pro Asn Ala Asn Lys Ile Arg
        115                 120                 125

Glu Ala Leu Ala Gln Thr His Ser Ala Ile Ala Val Ile Ile Gly Ile
    130                 135                 140

Lys Asp Leu Asp Ala Phe Arg His Tyr Asp Gly Arg Thr Ile Ile Gln
145                 150                 155                 160

Arg Asp Asn Gly Tyr Gln Pro Asn Tyr His Ala Val Asn Ile Val Gly
                165                 170                 175

Tyr Ser Asn Ala Gln Gly Val Asp Tyr Trp Ile Val Arg Asn Ser Trp
            180                 185                 190

Asp Thr Asn Trp Gly Asp Asn Gly Tyr Gly Tyr Phe Ala Ala Asn Ile
        195                 200                 205

Asp Leu Met Met Ile Glu Glu Tyr Pro Tyr Val Val Ile Leu
    210                 215                 220

<210>  4
<211>  129
<212>  PRT
<213>  Dermatophagoides pteronyssinus

<220>
<221>  MISC_FEATURE
<223>  Der p 2

<400>  4

Asp Gln Val Asp Val Lys Asp Cys Ala Asn His Glu Ile Lys Lys Val
1               5                   10                  15

Leu Val Pro Gly Cys His Gly Ser Glu Pro Cys Ile Ile His Arg Gly
            20                  25                  30

Lys Pro Phe Gln Leu Glu Ala Val Phe Glu Ala Asn Gln Asn Thr Lys
        35                  40                  45

Thr Ala Lys Ile Glu Ile Lys Ala Ser Ile Asp Gly Leu Glu Val Asp
    50                  55                  60

Val Pro Gly Ile Asp Pro Asn Ala Cys His Tyr Met Lys Cys Pro Leu
65                  70                  75                  80

Val Lys Gly Gln Gln Tyr Asp Ile Lys Tyr Thr Trp Asn Val Pro Lys
                85                  90                  95

Ile Ala Pro Lys Ser Glu Asn Val Val Val Thr Val Lys Val Met Gly
            100                 105                 110

Asp Asp Gly Val Leu Ala Cys Ala Ile Ala Thr His Ala Lys Ile Arg
        115                 120                 125

Asp

<210> 5
<211> 151
<212> PRT
<213> Apis mellifera

<220>
<221> MISC_FEATURE
<223> PLA2

<400> 5

His Gly Trp Gln Ile Arg Asp Arg Ile Gly Asp Asn Glu Leu Glu Glu
1               5                   10                  15

Arg Ile Ile Tyr Pro Gly Thr Leu Trp Cys Gly His Gly Asn Lys Ser
            20                  25                  30

Ser Gly Pro Asn Glu Leu Gly Arg Phe Lys His Thr Asp Ala Cys Cys
        35                  40                  45

Arg Thr His Asp Met Cys Pro Asp Val Met Ser Ala Gly Glu Ser Lys
    50                  55                  60

His Gly Leu Thr Asn Thr Ala Ser His Thr Arg Leu Ser Cys Asp Cys
65                  70                  75                  80

Asp Asp Lys Phe Tyr Asp Cys Leu Lys Asn Ser Ala Asp Thr Ile Ser
                85                  90                  95

Ser Tyr Phe Val Gly Lys Met Tyr Phe Asn Leu Ile Asp Thr Lys Cys
            100                 105                 110

Tyr Lys Leu Glu His Pro Val Thr Gly Cys Gly Glu Arg Thr Glu Gly
        115                 120                 125

Arg Cys Leu His Tyr Thr Val Asp Lys Ser Lys Pro Lys Val Tyr Gln
    130                 135                 140

Trp Phe Asp Leu Arg Lys Tyr
145                 150

<210> 6
<211> 70
<212> PRT
<213> felis domesticus

<220>
<221> MISC_FEATURE
<223> Fel D 1 chain 1

<400> 6

Glu Ile Cys Pro Ala Val Lys Arg Asp Val Asp Leu Phe Leu Thr Gly
1               5                   10                  15

Thr Pro Asp Glu Tyr Val Glu Gln Val Ala Gln Tyr Lys Ala Leu Pro
            20                  25                  30

Val Val Leu Glu Asn Ala Arg Ile Leu Lys Asn Cys Val Asp Ala Lys
        35                  40                  45

Met Thr Glu Glu Asp Lys Glu Asn Ala Leu Ser Leu Leu Asp Lys Ile
    50                  55                  60

Tyr Thr Ser Pro Leu Cys
65                  70

<210> 7
<211> 92
<212> PRT
<213> felis domesticus

<220>
<221> MISC_FEATURE
<223> Fel d 1 chain 2

<400> 7

Val Lys Met Ala Glu Thr Cys Pro Ile Phe Tyr Asp Val Phe Phe Ala
1               5                   10                  15

Val Ala Asn Gly Asn Glu Leu Leu Leu Asp Leu Ser Leu Thr Lys Val
            20                  25                  30

Asn Ala Thr Glu Pro Glu Arg Thr Ala Met Lys Lys Ile Gln Asp Cys
        35                  40                  45

Tyr Val Glu Asn Gly Leu Ile Ser Arg Val Leu Asp Gly Leu Val Met
    50                  55                  60

Thr Thr Ile Ser Ser Ser Lys Asp Cys Met Gly Glu Ala Val Gln Asn
65                  70                  75                  80

Thr Val Glu Asp Leu Lys Leu Asn Thr Leu Gly Arg
                85                  90

<210>  8
<211>  162
<212>  PRT
<213>  felis domesticus

<220>
<221>  MISC_FEATURE
<223>  Fel d 1 chain 1+chain 2

<400>  8

Glu Ile Cys Pro Ala Val Lys Arg Asp Val Asp Leu Phe Leu Thr Gly
1               5                   10                  15

Thr Pro Asp Glu Tyr Val Glu Gln Val Ala Gln Tyr Lys Ala Leu Pro
            20                  25                  30

Val Val Leu Glu Asn Ala Arg Ile Leu Lys Asn Cys Val Asp Ala Lys
        35                  40                  45

Met Thr Glu Glu Asp Lys Glu Asn Ala Leu Ser Leu Leu Asp Lys Ile
    50                  55                  60

Tyr Thr Ser Pro Leu Cys Val Lys Met Ala Glu Thr Cys Pro Ile Phe
65                  70                  75                  80

Tyr Asp Val Phe Phe Ala Val Ala Asn Gly Asn Glu Leu Leu Leu Asp
                85                  90                  95

Leu Ser Leu Thr Lys Val Asn Ala Thr Glu Pro Glu Arg Thr Ala Met
            100                 105                 110

Lys Lys Ile Gln Asp Cys Tyr Val Glu Asn Gly Leu Ile Ser Arg Val
        115                 120                 125

Leu Asp Gly Leu Val Met Thr Thr Ile Ser Ser Ser Lys Asp Cys Met
    130                 135                 140

Gly Glu Ala Val Gln Asn Thr Val Glu Asp Leu Lys Leu Asn Thr Leu
145                 150                 155                 160

Gly Arg

<210> 9
<211> 188
<212> PRT
<213> artificial sequence

<220>
<223> HIS-TAT-Fel D 1 (2+1)

<400> 9

Met Arg Gly Ser His His His His His His Gly Ser Gly Tyr Gly Arg
1               5                   10                  15

Lys Lys Arg Arg Gln Arg Arg Arg Gly Ser Val Lys Met Ala Glu Thr
            20                  25                  30

Cys Pro Ile Phe Tyr Asp Val Phe Phe Ala Val Ala Asn Gly Asn Glu
        35                  40                  45

Leu Leu Leu Asp Leu Ser Leu Thr Lys Val Asn Ala Thr Glu Pro Glu
    50                  55                  60

Arg Thr Ala Met Lys Lys Ile Gln Asp Cys Tyr Val Glu Asn Gly Leu
65                  70                  75                  80

Ile Ser Arg Val Leu Asp Gly Leu Val Met Thr Thr Ile Ser Ser Ser
                85                  90                  95

Lys Asp Cys Met Gly Glu Ala Val Gln Asn Thr Val Glu Asp Leu Lys
            100                 105                 110

Leu Asn Thr Leu Gly Arg Glu Ile Cys Pro Ala Val Lys Arg Asp Val
        115                 120                 125

Asp Leu Phe Leu Thr Gly Thr Pro Asp Glu Tyr Val Glu Gln Val Ala
    130                 135                 140

Gln Tyr Lys Ala Leu Pro Val Val Leu Glu Asn Ala Arg Ile Leu Lys
145                 150                 155                 160

Asn Cys Val Asp Ala Lys Met Thr Glu Glu Asp Lys Glu Asn Ala Leu
                165                 170                 175

Ser Leu Leu Asp Lys Ile Tyr Thr Ser Pro Leu Cys
            180                 185

<210> 10
<211> 177
<212> PRT
<213> artificial sequence

<220>
<223>  HIS-TAT-PLA2

<400>  10

Met Arg Gly Ser His His His His His His Gly Ser Gly Tyr Gly Arg
1               5                   10                  15

Lys Lys Arg Arg Gln Arg Arg Arg Gly Ser His Gly Trp Gln Ile Arg
            20                  25                  30

Asp Arg Ile Gly Asp Asn Glu Leu Glu Glu Arg Ile Ile Tyr Pro Gly
        35                  40                  45

Thr Leu Trp Cys Gly His Gly Asn Lys Ser Ser Gly Pro Asn Glu Leu
    50                  55                  60

Gly Arg Phe Lys His Thr Asp Ala Cys Cys Arg Thr His Asp Met Cys
65                  70                  75                  80

Pro Asp Val Met Ser Ala Gly Glu Ser Lys His Gly Leu Thr Asn Thr
                85                  90                  95

Ala Ser His Thr Arg Leu Ser Cys Asp Cys Asp Asp Lys Phe Tyr Asp
            100                 105                 110

Cys Leu Lys Asn Ser Ala Asp Thr Ile Ser Ser Tyr Phe Val Gly Lys
        115                 120                 125

Met Tyr Phe Asn Leu Ile Asp Thr Lys Cys Tyr Lys Leu Glu His Pro
    130                 135                 140

Val Thr Gly Cys Gly Glu Arg Thr Glu Gly Arg Cys Leu His Tyr Thr
145                 150                 155                 160

Val Asp Lys Ser Lys Pro Lys Val Tyr Gln Trp Phe Asp Leu Arg Lys
                165                 170                 175

Tyr


<210>  11
<211>  12
<212>  PRT
<213>  HIV


<220>
<221>  MISC_FEATURE
<223>  aa 46 to 57 of TAT

<400>  11

Gly Tyr Gly Arg Lys Lys Arg Arg Gln Arg Arg Arg
1               5                   10

<210>  12
<211>  34
<212>  PRT
<213>  Herpes virus

<400>  12

Asp Ala Ala Thr Ala Thr Arg Gly Arg Ser Ala Ala Ser Arg Pro Thr
1               5                   10                  15

Glu Arg Pro Arg Ala Pro Ala Arg Ser Ala Ser Arg Pro Arg Arg Pro
            20                  25                  30

Val Glu

<210>  13
<211>  16
<212>  PRT
<213>  Antennapedia

<400>  13

Arg Gln Ile Lys Ile Trp Phe Gln Asn Arg Arg Met Lys Trp Lys Lys
1               5                   10                  15